# EUROPÄISCHE PATENTSCHRIFT

(11) **EP 2 683 707 B1**
(45) Veröffentlichungstag und Bekanntmachung des Hinweises auf die Patenterteilung: **13.05.2015**
(21) Anmeldenummer: 12707090.2
(22) Anmeldetag: 01.03.2012
(51) Int. Cl.: C07D 307/52, C07C 213/02

(54) **VERFAHREN ZUR HERSTELLUNG VON ALKANOLAMINEN DURCH HOMOGEN-KATALYSIERTE ALKOHOLAMINIERUNG**
METHOD FOR PRODUCING ALKANOL AMINES OBTAINED BY HOMOGENEOUSLY CATALYZED ALCOHOL AMINATION
PROCÉDÉ DE PRÉPARATION D'ALCANOLAMINES PAR AMINATION D'ALCOOLS SOUS CATALYSE HOMOGÈNE

(30) Priorität: 08.03.2011 EP 11157348
(43) Veröffentlichungstag der Anmeldung: 15.01.2014
(73) Patentinhaber: BASF SE, 67056 Ludwigshafen (DE)
(72) Erfinder: SCHAUB, Thomas, 67433 Neustadt (DE); BUSCHHAUS, Boris, 68161 Mannheim (DE); BRINKS, Marion Kristina, 68165 Mannheim (DE); SCHELWIES, Mathias, 69115 Heidelberg (DE); PACIELLO, Rocco, 67098 Bad Dürkheim (DE); MELDER, Johann-Peter, 67459 Böhl-Iggelheim (DE); MERGER, Martin, 67227 Frankenthal (DE)
(86) Internationale Anmeldenummer: PCT/EP2012/053583
(87) Internationale Veröffentlichungsnummer: WO 2012/119928

(56) Entgegenhaltungen:
- EP-A1- 0 234 401
- WO-A1-2010/018570
- TILLACK A ET AL: "A novel ruthenium-catalyzed amination of primary and secondary alcohols", TETRAHEDRON LETTERS, ELSEVIER, AMSTERDAM, NL, Bd. 47, Nr. 50, 11. Dezember 2006 (2006-12-11), Seiten 8881-8885, XP025003579, ISSN: 0040-4039, DOI: 10.1016/J.TETLET.2006.10.042 [gefunden am 2006-12-11]
- IMM S ET AL: "Improved Ruthenium-Catalyzed Amination of Alcohols with Ammonia: Synthesis of Diamines and Amino Esters", ANGEWANDTE CHEMIE. INTERNATIONAL EDITION, WILEY VCH VERLAG, WEINHEIM, Bd. 50, Nr. 33, 8. August 2011 (2011-08-08), Seiten 7599-7603, XP002664616, ISSN: 1433-7851, DOI: 10.1002/ANIE.201103199 [gefunden am 2011-07-05]

## Beschreibung

Die vorliegende Erfindung betrifft ein Verfahren zur Herstellung von Alkanolaminen durch homogen-katalysierte Alkoholaminierung von Diolen mit Ammoniak unter Wasserabspaltung in Gegenwart eines Komplexkatalysators, der mindestens ein Element ausgewählt aus den Gruppen 8, 9 und 10 des Periodensystems sowie mindestens einen Phosphordonorliganden enthält.

Alkanolamine sind Verbindungen, die eine primäre Aminogruppe (-NH₂) und eine Hydroxylgruppe (-OH) aufweisen.

Alkanolamine sind wertvolle Produkte mit einer Vielzahl unterschiedlicher Verwendungen, beispielsweise als Lösungsmittel, Stabilisatoren, zur Synthese von ChelatBildnern, als Edukte zur Herstellung von Kunstharzen, Arzneimitteln, Inhibitoren, Korrosionsschutzmitteln, Polyurethanen, als Härter für Epoxyharze, als grenzflächenaktive Substanzen und zur Gaswäsche.

Die Aminierung von Diolen mit sekundären Aminen mittels homogenen Iridium- und Rutheniumkatalysatoren zu Aminoalkoholen und linearen Diaminen mit tertiären Aminogruppen ist beispielsweise in EP 239 934; J. A. Marsella, J. Org. Chem. 1987, 52, 467-468; US 4,855,425; K.-T. Huh, Bull. Kor. Chem. Soc. 1990, 11, 45-49; N. Andrushko, V. Andrushko, P. Roose, K. Moonen, A. Börner, ChemCatChem, 2010, 2, 640-643 und S. Bähn, A. Tillack, S. Imm, K. Mevius, D. Michalik, D. Hollmann, L. Neubert, M. Beller, ChemSusChem 2009, 2, 551-557 beschrieben. Die Aminierung wird in diesen Arbeiten bei 100-180°C durchgeführt.

J. A. Marsella, J. Organomet. Chem. 1991, 407, 97-105 und B. Blank, S. Michlik, R. Kempe, Adv. Synth. Catal. 2009, 351, 2903-2911; G. Jenner, G. Bitsi, J. Mol. Cat, 1988, 45, 165-168; Y. Z. Youn, D. Y. Lee, B. W. Woo, J. G. Shim, S. A. Chae, S. C. Shim, J. Mol. Cat, 1993, 79, 39-45; K. I. Fujita, R. Yamaguchi, Synlett, 2005, 4, 560-571; K.I. Fujii, R. Yamaguchi, Org. Lett. 2004, 20, 3525-3528; K. I. Fujita, K. Yamamoto, R. Yamaguchi, Org. Lett. 2002, 16, 2691-2694; A. Nova, D. Balcells, N. D. Schley, G. E. Dobereiner, R. H. Crabtree, O. Eisenstein, Organometallics DOI: 10.1021/om101015u; und M. H. S. A. Hamid, C. L. Allen, G. W. Lamb, A. C. Maxwell, H. C. Maytum, A. J. A. Watson, J. M. J. Williams, J. Am. Chem. Soc. 2009, 131, 1766-1774 und O. Saidi, A. J. Blacker, G. W. Lamb, S. P. Marsden, J. E. Taylor, J. M. J. Williams, Org. Proc. Res. Dev. 2010, 14, 1046-1049 beschreiben die Aminierung von Diolen mit primären Aminen unter Verwendung von homogen-gelösten Ruthenium- und Iridium-basierten Übergangsmetallkatalysatoren. Hierbei bilden sich jedoch die zyklischen Verbindungen und nicht die gewünschten Alkanolamine. Die wirtschaftlich attraktive Aminierung von Diolen mit Ammoniak zu Alkanolaminen ist mit diesen Systemen nicht beschrieben.

EP 0 234 401 A1 beschreibt die Umsetzung von Ethylenglykol mit Ammoniak in Gegenwart einer Rutheniumcarbonylverbindung. Bei dem in EP 0 234 401 A1 beschriebene Verfahren bilden sich unter anderem das Monoaminierungsprodukt (Monoethanolamin). Daneben entstehen allerdings in großen Mengen die sekundären und tertiären Amine (Di- und Triethanolamin) und zyklische Produkte (N-(Hydroxyethyl)piperazin und N,N'-Bis(hydroxyethyl)piperazin) als Nebenprodukte.

Alle vorstehend beschriebenen Verfahren zur Umsetzung von Diolen haben den Nachteil, dass sich neben den gewünschten Alkanolaminen größtenteils unerwünschte sekundäre, tertiäre und zyklische Amine bilden.

Aufgabe der vorliegenden Erfindung ist es, ein Verfahren zur Herstellung von Alkanolaminen durch Alkoholaminierung von Diolen mit Ammoniak unter Wasserabspaltung bereitzustellen.

Gelöst wird die Aufgabe durch ein Verfahren zur Herstellung von Alkanolaminen, die eine primäre Aminogruppe (-NH₂) und eine Hydroxylgruppe (-OH) aufweisen, durch Alkoholaminierung von Diolen, die zwei Hydroxylgruppen (-OH) aufweisen, mit Ammoniak unter Wasserabspaltung, wobei die Reaktion homogen-katalysiert in Gegenwart mindestens eines Komplexkatalysators, der mindestens ein Element ausgewählt aus den Gruppen 8, 9 und 10 des Periodensystems sowie mindestens einen Phosphordonorliganden enthält, durchgeführt wird.

Überraschend wurde festgestellt, dass sich mit den im erfindungsgemäßen Verfahren eingesetzten Komplexkatalysatoren, die mindestens ein Element ausgewählt aus der Gruppe 8, 9 und 10 des Periodensystems sowie mindestens einen Phosphordonorliganden enthalten, Alkanolamine durch die homogen-katalysierte Aminierung von Diolen mit Ammoniak unter Wasserabspaltung erhalten lassen. Das erfindungsgemäße Verfahren hat den Vorteil, dass es Alkanolamine gegenüber den im Stand der Technik beschriebenen Verfahren in deutlich verbesserten Ausbeuten liefert. Darüber hinaus wird die Bildung unerwünschter Nebenprodukte wie sekundärer und tertiärer Amine sowie zyklischer Amine weitestgehend vermieden.

### Edukte

Im erfindungsgemäßen Verfahren werden Edukte eingesetzt, die zwei Hydroxylgruppen aufweisen.

Als Edukte sind praktisch alle Diole geeignet, die die vorstehend genannten Voraussetzungen erfüllen. Die Diole können geradkettig, verzweigt oder zyklisch sein. Die Alkohole können darüber hinaus Substituenten tragen, die sich unter den Reaktionsbedingungen der Alkoholaminierung inert verhalten, beispielsweise Alkoxy, Alkenyloxy, Alkylamino, Dialkylamino und Halogene (F, Cl, Br, I).

Geeignete Edukte, die im erfindungsgemäßen Verfahren eingesetzt werden können, sind beispielsweise Diole, die eine funktionelle Gruppe der Formel (-CH₂-OH) und eine weitere Hydroxylgruppe (-OH) aufweisen.

Darüber hinaus sind Diole geeignet, die zwei funktionelle Gruppen der Formel (-CH₂-OH) aufweisen.

Als Edukte können alle bekannten Diole eingesetzt werden. Bevorzugt sind Diole, die mindestens eine funktionelle Gruppe der Formel (-CH₂-OH) aufweisen. Mehr bevorzugt sind Diole, die zwei funktionelle Gruppen der Formeln (-CH₂-OH) aufweisen. Beispiele für Diole, die als Edukte in das erfindungsgemäße Verfahren eingesetzt werden können, sind 1,2-Ethandiol (Ethylenglykol), 1,2-Propandiol (1,2-Propylenglykol), 1,3-Propandiol (1,3-Propylenglykol), 1,4-Butandiol (1,4-Butylenglykol), 1,2-Butandiol (1,2-Butylenglykol), 2,3-Butandiol, 2-Methyl-1,3-propandiol, 2,2-Dimethyl-1,3-propandiol (Neopentylglykol), 1,5-Pentandiol, 1,2-Pentandiol, 1,6-Hexandiol, 1,2-Hexandiol, 1,7-Heptandiol, 1,2-Heptandiol, 1,8-Oktandiol, 1,2-Oktandiol, 1,9-Nonandiol, 1,2-Nonandiol, 2,4-Dimethyl-2,5-hexandiol, Hydroxypivalinsäureneopentylglykolester, Diethylenglykol, Triethylenglykol, 2-Buten-1,4-diol, 2-Butin-1,4-diol, Poylyethlenglykole, Polypropylenglykole wie 1,2-Polypropylenglykol und 1,3 Polypropylenglykol, Polytetrahydrofuran, Diethanolamin, 1,4-Bis-(2-hydroxyethyl)piperazin, Düsopropanolamin, N-Butyldiethanolamin, N-Methyldiethanolamin, 1,10-Decandiol, 1,12-Dodecandiol, 2,5-(Dimethanol)-furan und C₃₆-Diol (Gemisch aus Isomeren von Alkoholen der Summenformel C₃₆H₇₄O₂).

### 2,5-(Dimethanol)-furan wird auch als 2,5-Bis(hydroxymethyl)furan bezeichnet.

Weiter sind Diole der allgemeinen Formeln (XXXI), (XXXII) und (XXXIII) geeignet: 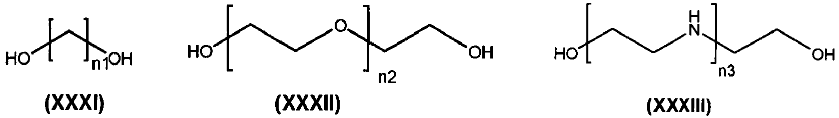 wobei
- n1: ist 2 - 30;
- n2: ist 1 - 30 und
- n3: ist 1 - 30.

Bevorzugt sind Diole, die zwei funktionelle Gruppen der Formel (-CH₂-OH) aufweisen.

Insbesondere bevorzugte Diole sind 1,2-Ethandiol (Ethylenglykol), 1,2-Propandiol (1,2-Propylenglykol), 1,3-Propandiol (1,3-Propylenglykol), 1,4-Butandiol (1,4-Butylenglykol), 1,2-Butandiol (1,2-Butylenglykol), 2,3-Butandiol, 2-Methyl-1,3-propandiol, 2,2-Dimethyl-1,3-propandiol (Neopentylglykol), Diethylenglykol, Triethylenglykol, Polyethylenglykole, Polypropylenglykole wie 1,2-Polypropylenglykol und 1,3-Polypropylenglykol, Polytetrahydrofuran, 2,5-(Dimethanol)-furan und Diethanolamin.

### Komplexkatalysator

Im erfindungsgemäßen Verfahren wird mindestens ein Komplexkatalysator, der mindestens ein Element ausgewählt aus den Gruppen 8, 9 und 10 des Periodensystems (Nomenklatur gemäß IUPAC) sowie mindestens einen Donorliganden enthält, eingesetzt. Die Elemente der Gruppe 8, 9 und 10 des Periodensystems umfassen Eisen, Cobalt, Nickel, Ruthenium, Rhodium, Palladium, Osmium, Iridium und Platin. Bevorzugt sind Komplexkatalysatoren, die mindestens ein Element ausgewählt aus Ruthenium und Iridium enthalten.

In einer Ausführungsform wird das erfindungsgemäße Verfahren homogen-katalysiert in Gegenwart mindestens eines Komplexkatalysators der allgemeinen Formel (I) durchgeführt: 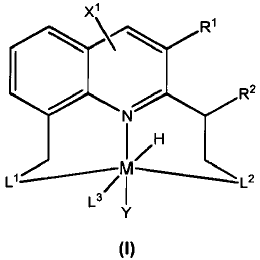 in der
- L¹ und L²: unabhängig voneinander Phosphin (PR^{a}R^{b}), Amin (NR^{a}R^{b}), Sulfid, SH, Sulfoxid (S(=O)R), C₅-C₁₀-Heteroaryl enthaltend mindestens ein Heteroatom ausgewählt aus Stickstoff (N), Sauerstoff (O) und Schwefel (S), Arsin (AsR^{a}R^{b}), Stiban (SbR^{a}R^{b}) oder N-heterozyklische Carbene der Formel (II) oder (III): 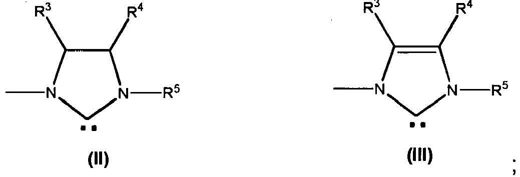
- L³: einzähniger Zweielektronendonor ausgewählt aus der Gruppe Kohlenmonoxid (CO), PR^{a}R^{b}R^{c}, NO⁺, AsR^{a}R^{b}R^{c}, SbR^{a}R^{b}R^{c}, SR^{a}R^{b}, Nitril (RCN), Isonitril (RNC), Stickstoff (N₂), Phosphortrifluorid (PF₃), Kohlenstoffmonosulfid (CS), Pyridin, Thiophen, Tetrahydrothiophen und N-heterozyklische Carbene der Formel (II) oder (III);
- R¹ und R²: beide Wasserstoff oder gemeinsam mit den Kohlenstoffatomen, an die sie gebunden sind, ein Phenylring, der zusammen mit der Chinolinyleinheit der Formel I eine Acridinyleinheit bildet;
- R, R^{a}, R^{b}, R^{c}, R³, R⁴, und R⁵: unabhängig voneinander unsubstituiertes oder zumindest monosubstituiertes C₁-C₁₀-Alkyl, C₃-C₁₀-Cycloalkyl, C₃-C₁₀-Heterocyclyl enthaltend mindestens ein Heteroatom ausgewählt aus N, O und S, C₅-C₁₀-Aryl oder C₅-C₁₀-Heteroaryl enthaltend mindestens ein Heteroatom ausgewählt aus N, O und S,
wobei die Substituenten ausgewählt sind aus der Gruppe bestehend aus: F, Cl, Br, OH, CN, NH₂ und C₁-C₁₀-Alkyl;
- Y: monoanionischer Ligand ausgewählt aus der Gruppe H, F, Cl, Br, I, OCOR, OCOCF₃, OSO₂R, OSO₂CF₃, CN, OH, OR und N(R)₂ oder neutrale Moleküle ausgewählt aus der Gruppe NH₃, N(R)₃ und R₂NSO₂R;
- X¹: ein, zwei, drei, vier, fünf, sechs, oder sieben Substituenten an einem oder mehreren Atomen der Acridinyleinheit oder ein, zwei, drei, vier oder fünf Substituenten an einem oder mehreren Atomen der Chinolinyleinheit,
wobei X¹ unabhängig voneinander ausgewählt ist aus der Gruppe bestehend aus Wasserstoff, F, Cl, Br, I, OH, NH₂, NO₂, -NC(O)R, C(O)NR₂, -OC(O)R, -C(O)OR, CN und Boranderivaten, die aus dem Katalysator der Formel I durch Reaktion mit NaBH₄ erhältlich sind und unsubstituiertem oder zumindest monosubstituiertem C₁-C₁₀-Alkoxy, C₁-C₁₀-Alkyl, C₃-C₁₀-Cycloalkyl, C₃-C₁₀-Heterocyclyl enthaltend mindestens ein Heteroatom ausgewählt aus N, O und S, C₅-C₁₀-Aryl und C₅-C₁₀-Heteroaryl enthaltend mindestens ein Heteroatom ausgewählt aus N, O und S,
wobei die Substituenten ausgewählt sind aus der Gruppe bestehend aus: F, Cl, Br, OH, CN, NH₂ und C₁-C₁₀-Alkyl;
und
- M: Eisen, Cobalt, Nickel, Ruthenium, Rhodium, Palladium, Osmium, Iridium oder Platin
bedeuten.

Hierbei ist darauf hinzuweisen, dass der Komplexkatalysator der Formel (I) für den Fall, dass Y ein neutrales Molekül aus der Gruppe NH₃, NR₃, R₂NSO₂R ist und M ausgewählt ist aus der Gruppe Ruthenium, Nickel, Palladium und Eisen, eine positive Ladung trägt.

In einer bevorzugten Ausführungsform wird das erfindungsgemäßen Verfahrens in Gegenwart mindestens eines homogen gelösten Komplexkatalysators der Formel (I) durchgeführt, wobei die Substituenten folgende Bedeutung haben:
- L¹ und L²,: unabhängig voneinander PR^{a}R^{b}, NR^{a}R^{b}, Sulfid, SH, S(=O)R, C₅-C₁₀-Heteroaryl enthaltend mindestens ein Heteroatom ausgewählt aus N, O und S;
- L³: einzähniger Zweielektronendonor, ausgewählt aus der Gruppe CO, PR^{a}R^{b}R^{c}, NO⁺, RCN, RNC, N₂, PF₃, CS, Pyridin, Thiophen und Tetrahydrothiophen;
- R¹ und R²: beide Wasserstoff oder gemeinsam mit den Kohlenstoffatomen, an die sie gebunden sind ein Phenylring, der zusammen mit der Chinolinyleinheit der Formel (I) eine Acridinyleinheit bildet;
- R, R^{a}, R^{b}, R^{c}, R³, R⁴, und R⁵: unabhängig voneinander unsubstituiertes C₁-C₁₀-Alkyl, C₃-C₁₀-Cycloalkyl, C₃-C₁₀-Heterocyclyl enthaltend mindestens ein Heteroatom ausgewählt aus N, O und S, C₅-C₁₀-Aryl oder C₅-C₁₀-Heteroaryl enthaltend mindestens ein Heteroatom ausgewählt aus N, O und S;
- Y: monoanionischer Ligand ausgewählt aus der Gruppe H, F, Cl, Br, OCOR, OCOCF₃, OSO₂R, OSO₂CF₃, CN, OH, OR oder N(R)₂;
- X¹: ein, zwei, drei, vier, fünf, sechs, oder sieben Substituenten an einem oder mehreren Atomen der Acridinyleinheit oder ein, zwei, drei, vier oder fünf Substituenten an einem oder mehreren Atomen der Chinolinyleinheit,
wobei X¹ unabhängig voneinander ausgewählt ist aus der Gruppe bestehend aus Wasserstoff, F, Cl, Br, I, OH, NH₂, NO₂, -NC(O)R, C(O)NR₂, -OC(O)R, -C(O)OR, CN und Boranderivaten, die aus dem Katalysator der Formel (I) durch Reaktion mit NaBH₄ erhältlich sind und unsubstituiertem C₁-C₁₀-Alkoxy, C₁-C₁₀-Alkyl, C₃-C₁₀-Cycloalkyl, C₃-C₁₀-Heterocyclyl enthaltend mindestens ein Heteroatom ausgewählt aus N, O und S, C₅-C₁₀-Aryl und C₅-C₁₀-Heteroaryl enthaltend mindestens ein Heteroatom ausgewählt aus N, O und S;
und
- M: Ruthenium oder Iridium.

In einer weiteren bevorzugten Ausführungsform wird das erfindungsgemäße Verfahren in Gegenwart mindestens eines homogen gelösten Komplexkatalysator durchgeführt, wobei R¹ und R² beide Wasserstoff sind und der Komplexkatalysator ein Katalysator der Formel (IV) ist: 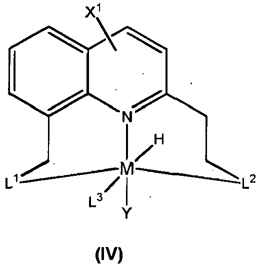 und X¹, L¹, L², L³ und Y die vorstehend genannten Bedeutungen aufweisen.

In einer weiteren bevorzugten Ausführungsform wird das erfindungsgemäßen Verfahrens in Gegenwart mindestens eines homogen gelösten Komplexkatalysator durchgeführt, wobei R¹ und R² gemeinsam mit den Kohlenstoffatomen, an die sie gebunden sind ein Phenylring bilden, der zusammen mit der Chinolinyleinheit der Formel (I) eine Acridinyleinheit bildet und der Komplexkatalysator ein Katalysator der Formel (V) ist: 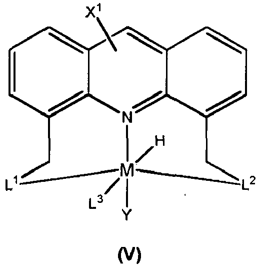 und X¹, L¹, L², L³ und Y die vorstehend genannten Bedeutungen aufweisen.

Nachfolgend werden exemplarisch einige Komplexkatalysatoren (Formeln (VI), (VII), (VIII), (IX), (X), (XI), (XII) und (XIII)) aufgeführt, die im erfindungsgemäßen Verfahren eingesetzt werden können:

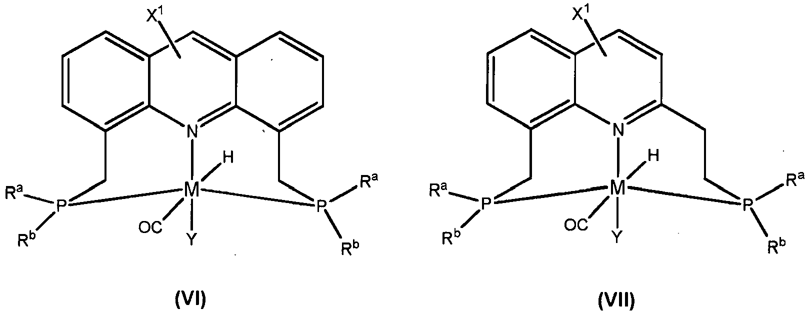

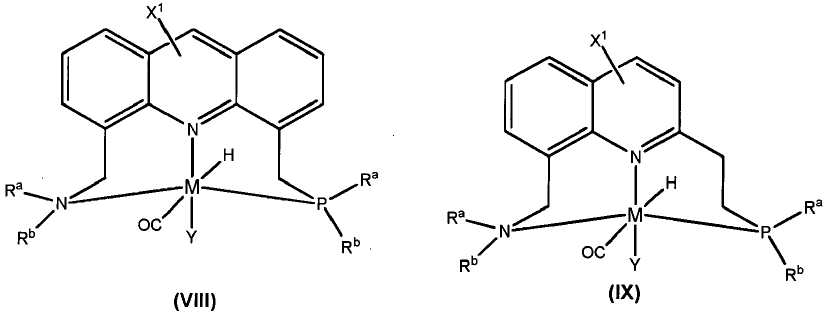

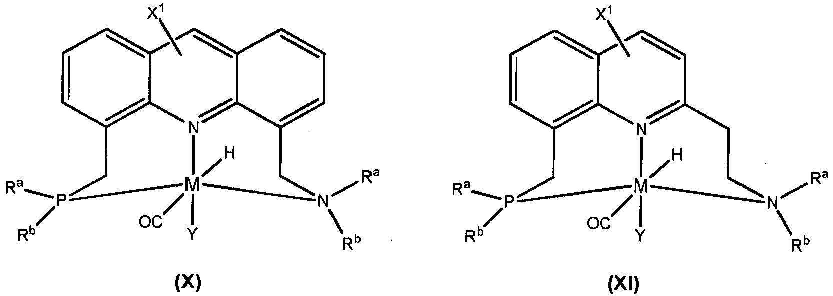

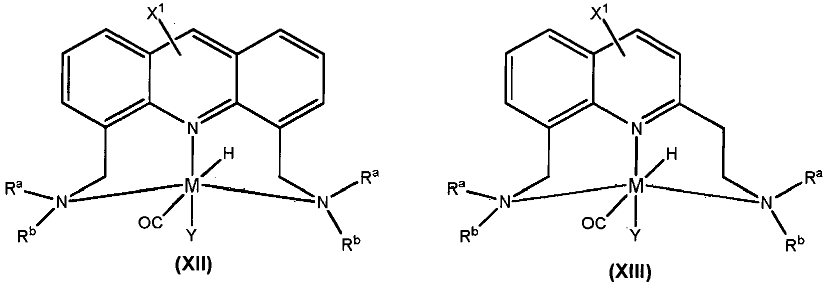

In einer weiteren bevorzugten Ausführungsform wird das erfindungsgemäße Verfahrens in Gegenwart mindestens eines Komplexkatalysator aus der Gruppe der Katalysatoren der Formel (VI), (VII), (VIII), (IX), (X), (XI), (XII) und (XIII) durchgeführt, wobei
- R^{a} und R^{b}: unabhängig voneinander unsubstituiertes oder zumindest monosubstituiertes C₁-C₁₀)-Alkyl, C₃-C₁₀-Cycloalkyl, C₃-C₁₀-Heterocyclyl enthaltend mindestens ein Heteroatom ausgewählt aus N, O und S, C₅-C₁₀-Aryl oder C₅-C₁₀-Heteroaryl enthaltend mindestens ein Heteroatom ausgewählt aus N, O und S,
wobei die Substituenten ausgewählt sind aus der Gruppe bestehend aus: F, Cl, Br, OH, CN, NH₂ und C₁-C₁₀-Alkyl;
- Y: monoanionischer Ligand ausgewählt aus der Gruppe H, F, Cl, Br, OCOR, OCOCF₃, OSO₂R, OSO₂CF₃, CN, OH, OR, N(R)₂;
- R: unsubstituiertes oder zumindest monosubstituiertes C₁-C₁₀-Alkyl, C₃-C₁₀-Cycloalkyl, C₃-C₁₀-Heterocyclyl enthaltend mindestens ein Heteroatom ausgewählt aus N, O und S, C₅-C₁₀-Aryl, C₅-C₁₀-Heteroaryl enthaltend mindestens ein Heteroatom ausgewählt aus N, O und S,
wobei die Substituenten ausgewählt sind aus der Gruppe bestehend aus: F, Cl, Br, OH, CN, NH₂ und C₁-C₁₀-Alkyl;
- X¹: ein, zwei oder drei, Substituenten an einem oder mehreren Atomen der Acridinyleinheit oder ein oder zwei Substituenten an einem oder mehreren Atomen der Chinolinyleinheit,
wobei X¹ unabhängig voneinander ausgewählt ist aus der Gruppe bestehend aus Wasserstoff, F, Cl, Br, I, OH, NH₂, NO₂, -NC(O)R, C(O)NR₂, -OC(O)R, - C(O)OR, CN und Boranderivaten, die aus dem Katalysator der Formel I durch Reaktion mit NaBH₄ erhältlich sind und unsubstituiertem C₁-C₁₀-Alkoxy, C₁-C₁₀-Alkyl, C₃-C₁₀-Cycloalkyl, C₃-C₁₀-Heterocyclyl enthaltend mindestens ein Heteroatom ausgewählt aus N, O und S, C₅-C₁₀-Aryl und C₅-C₁₀-Heteroaryl enthaltend mindestens ein Heteroatom ausgewählt aus N, O und S;
und
- M: Ruthenium oder Iridium
bedeuten.

In einer weiteren bevorzugten Ausführungsform wird das erfindungsgemäße Verfahrens in Gegenwart mindestens eines Komplexkatalysator aus der Gruppe der Katalysatoren der Formel (VI), (VII), (VIII), (IX), (X), (XI), (XII) und (XIII) durchgeführt, wobei
- R^{a} und R^{b}: unabhängig voneinander Methyl, Ethyl, Isopropyl, tert.-Butyl, Cyclohexyl, Cyclopentyl, Phenyl oder Mesityl;
- Y: monoanionischer Ligand ausgewählt aus der Gruppe H, F, Cl, Br, OCOCH₃, OCOCF₃, OSO₂CF₃, CN und OH;
- X¹: ein Substituent an einem Atom der Acridinyleinheit oder ein Substituenten an einem Atom der Chinolinyleinheit,
wobei X¹ ausgewählt ist aus der Gruppe bestehend aus Wasserstoff, F, Cl, Br, OH, NH₂, NO₂, -NC(O)R, C(O)NR₂, -OC(O)R, -C(O)OR, CN und Boranderivaten, die aus dem Katalysator der Formel (I) durch Reaktion mit NaBH₄ erhältlich sind und unsubstituiertem C₁-C₁₀-Alkoxy, C₁-C₁₀-Alkyl, C₃-C₁₀-Cycloalkyl, C₃-C₁₀-Heterocyclyl enthaltend mindestens ein Heteroatom ausgewählt aus N, O und S, C₅-C₁₀-Aryl und C₅-C₁₀-Heteroaryl enthaltend mindestens ein Heteroatom ausgewählt aus N, O und S;
- M: Ruthenium oder Iridium
bedeuten.

In einer weiteren bevorzugten Ausführungsform wird das erfindungsgemäße Verfahrens in Gegenwart mindestens eines Komplexkatalysator aus der Gruppe der Katalysatoren der Formel (VI), (VII), (VIII), (IX), (X), (XI), (XII) und (XIII) durchgeführt, wobei
- R^{a} und R^{b}: unabhängig voneinander Methyl, Ethyl, Isopropyl, tert. Butyl, Cyclohexyl, Cyclopentyl, Phenyl oder Mesityl;
- Y: monoanionischer Ligand aus der Gruppe H, F, Cl, Br, I, OCOCH₃, OCOCF₃, OSO₂CF₃, CN und OH;
- X¹: Wasserstoff;
und
- M: Ruthenium oder Iridium
bedeuten.

In einer besonders bevorzugten Ausführungsform ist L³ Kohlenmonoxid (CO).

In einer besonders bevorzugten Ausführungsform wird das erfindungsgemäße Verfahren in Gegenwart eines Komplexkatalysators der Formel (XIVa) durchgeführt:

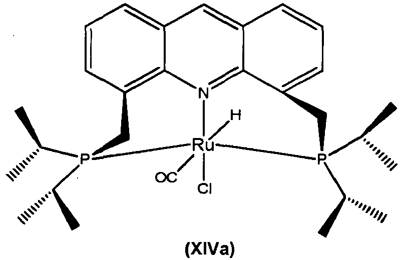

In einer insbesondere bevorzugten Ausführungsform wird das erfindungsgemäße Verfahren in Gegenwart eines Komplexkatalysators der Formel (XIVb) durchgeführt:

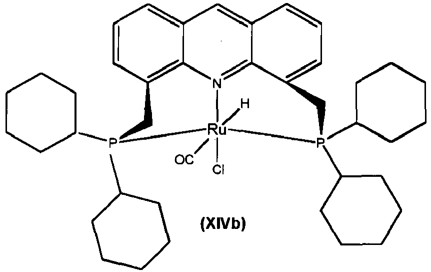

In einer weiteren besonders bevorzugten Ausführungsform wird das erfindungsgemäße Verfahrens in Gegenwart mindestens eines homogen gelösten Komplexkatalysator der Formel (XV) durchgeführt, in der R¹, R², R³, L¹, L² und L³ die vorstehend beschriebenen Bedeutungen haben.

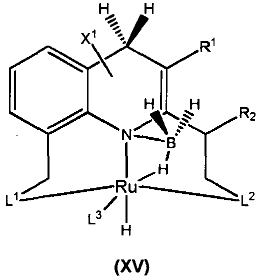

Komplexkatalysatoren der Formel (XV) sind erhältlich durch Umsetzung von Katalysatoren der Formel (I) mit Natriumborhydrid (NaBH₄). Die Umsetzung folgt dabei der allgemeinen Reaktionsgleichung:

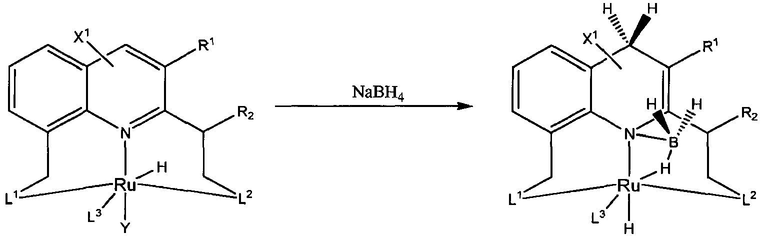

In einer weiteren besonders bevorzugten Ausführungsform wird das erfindungsgemäße Verfahrens in Gegenwart eines Komplexkatalysators der Formel (XVI) durchgeführt:

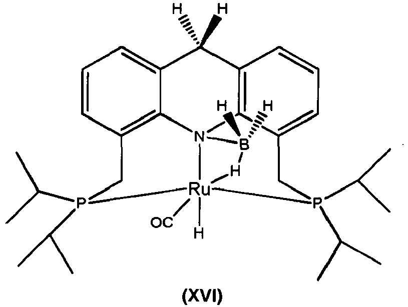

Das Boranderivat der Formel XVI ist gemäß folgender Reaktionsgleichung zugänglich:

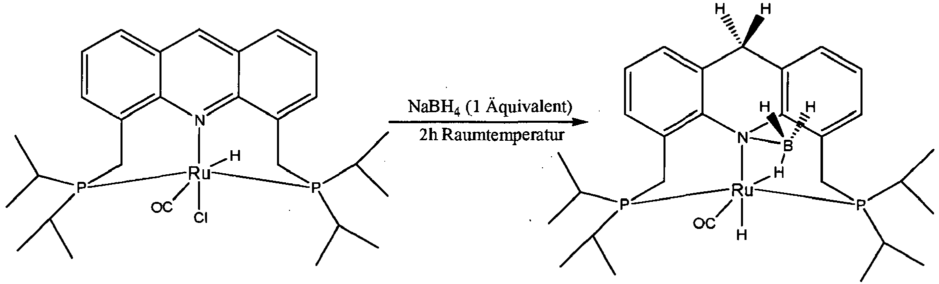

In einer weiteren Ausführungsform wird im erfindungsgemäßen Verfahren mindestens ein Komplexkatalysator, der mindestens ein Element ausgewählt aus den Gruppen 8, 9 und 10 des Periodensystems (Nomenklatur gemäß IUPAC) sowie mindestens einen Phosphordonorliganden der allgemeinen Formel (XXI) enthält, 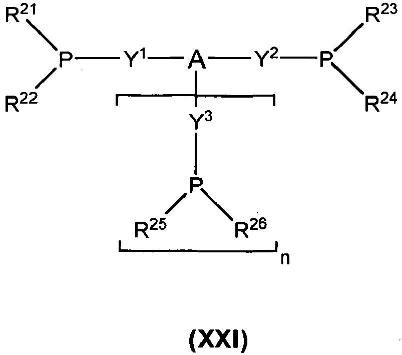 eingesetzt, wobei
- n: ist 0 oder 1;
- R²¹,: R²², R²³, R²⁴, R²⁵, R²⁶ sind unabhängig voneinander unsubstituiertes oder zumindest monosubstituiertes C₁-C₁₀-Alkyl, C₁-C₄-Alkyldiphenylphosphin (-C₁-C₄-Alkyl-P(Phenyl)₂), C₃-C₁₀-Cycloalkyl, C₃-C₁₀-Heterocyclyl enthaltend mindestens ein Heteroatom ausgewählt aus N, O und S, C₅-C₁₄-Aryl oder C₅-C₁₀-Heteroaryl enthaltend mindestens ein Heteroatom ausgewählt aus N, O und S,
wobei die Substituenten ausgewählt sind aus der Gruppe bestehend aus: F, Cl, Br, OH, CN, NH₂ und C₁-C₁₀-Alkyl;
- A: ist
i) eine Brückengruppe ausgewählt aus der Gruppe unsubstituiertes oder zumindest monosubstituiertes N, O, P, C₁-C₆-Alkan, C₃-C₁₀-Cycloalkan, C₃-C₁₀-Heterocycloalkan enthaltend mindestens ein Heteroatom ausgewählt aus N, O und S, C₅-C₁₄-Aromat und C₅-C₆-Heteroaromat enthaltend mindestens ein Heteroatom ausgewählt aus N, O und S,
   wobei die Substituenten ausgewählt sind aus der Gruppe bestehend aus: C₁-C₄-Alkyl, Phenyl, F, Cl, Br, OH, OR²⁷, NH₂, NHR²⁷ oder N(R²⁷)₂,
   wobei R²⁷ ausgewählt ist aus C₁-C₁₀-Alkyl und C₅-C₁₀-Aryl;
   oder
ii) eine Brückengruppe der Formel (XXII) oder (XXIII): 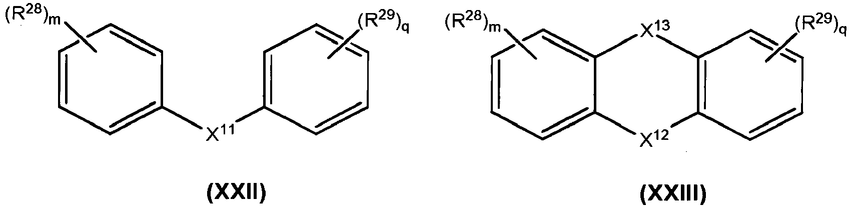
- m, q: sind unabhängig voneinander 0, 1, 2, 3 oder 4;
- R²⁸, R²⁹: sind unabhängig voneinander ausgewählt aus der Gruppe C₁-C₁₀-Alkyl, F, Cl, Br, OH, OR²⁷, NH₂, NHR²⁷ und N(R²⁷)₂,
wobei R²⁷ ausgewählt ist aus C₁-C₁₀-Alkyl und C₅-C₁₀-Aryl;
- X¹¹, X¹²: sind unabhängig voneinander NH, O oder S;
- X¹³: ist eine Bindung, NH, NR³⁰, O, S oder CR³¹R³²;
- R³⁰: ist unsubstituiertes oder zumindest monosubstituiertes C₁-C₁₀-Alkyl, C₃-C₁₀-Cycloalkyl, C₃-C₁₀-Heterocyclyl enthaltend mindestens ein Heteroatom ausgewählt aus N, O und S, C₅-C₁₄-Aryl oder C₅-C₁₀-Heteroaryl enthaltend mindestens ein Heteroatom ausgewählt aus N, O und S,
wobei die Substituenten ausgewählt sind aus der Gruppe bestehend aus: F, Cl, Br, OH, CN, NH₂ und C₁-C₁₀-Alkyl;
- R³¹ , R³²: sind unabhängig voneinander unsubstituiertes oder zumindest monosubstituiertes C₁-C₁₀-Alkyl, C₁-C₁₀-Alkoxy, C₃-C₁₀-Cycloalkyl, C₃-C₁₀Cycloalkoxy,C₃-C₁₀-Heterocyclyl enthaltend mindestens ein Heteroatom ausgewählt aus N, O und S, C₅-C₁₄-Aryl, C₅-C₁₄-Aryloxy oder C₅-C₁₀-Heteroaryl enthaltend mindestens ein Heteroatom ausgewählt aus N, O und S,
wobei die Substituenten ausgewählt sind aus der Gruppe bestehend aus: F, Cl, Br, OH, CN, NH₂ und C₁-C₁₀-Alkyl;
- Y¹, Y², Y³: sind unabhängig voneinander eine Bindung, unsubstituiertes oder zumindest monosubstituiertes Methylen, Ethylen, Trimethylen, Tetramethylen, Pentamethylen oder Hexamethylen,
wobei die Substituenten ausgewählt sind aus der Gruppe bestehend aus: F, Cl, Br, OH, OR²⁷, CN, NH₂, NHR²⁷, N(R²⁷)₂ und C₁-C₁₀-Alkyl,
wobei R²⁷ ausgewählt ist aus C₁-C₁₀-Alkyl und C₅-C₁₀-Aryl.

A ist erfindungsgemäß eine Brückengruppe. Für den Fall, dass A ausgewählt ist aus der Gruppe unsubstituiertes oder zumindest monosubstituiertes C₁-C₆-Alkan, C₃-C₁₀-Cycloalkan, C₃-C₁₀ Heterocycloalkan, C₅-C₁₄-Aromat und C₅-C₆-Heteroaromat und Brückengruppen der Formel (II) oder (III), sind für den Fall (n = 0) zwei Wasserstoffatome der Brückengruppe durch Bindungen zu den benachbarten Substituenten Y¹ und Y² ersetzt. Für den Fall (n = 1) sind drei Wasserstoffatome der Brückengruppe durch drei Bindungen zu den benachbarten Substituenten Y¹, Y² und Y³ ersetzt.

Für den Fall, dass A P (Phosphor) ist, bildet der Phosphor für den Fall (n=0) zwei Bindungen zu den benachbarten Substituenten Y¹ und Y² und eine Bindung zu einem Substituenten ausgewählt aus der Gruppe bestehend aus C₁-C₄-Alkyl und Phenyl aus. Für den Fall (n=1) bildet der Phosphor drei Bindungen zu den benachbarten Substituenten Y¹, Y² und Y³ aus.

Für den Fall, dass A N (Stickstoff) ist, bildet der Stickstoff für den Fall (n = 0) zwei Bindungen zu den benachbarten Substituenten Y¹ und Y² und eine Bindung zu einem Substituenten ausgewählt aus der Gruppe bestehend aus C₁-C₄-Alkyl und Phenyl aus. Für den Fall (n = 1) bildet der Stickstoff drei Bindungen zu den benachbarten Substituenten Y¹, Y² und Y³ aus.

Für den Fall, dass A O (Sauerstoff) ist, ist n = 0. Der Sauerstoff bildet zwei Bindungen zu den benachbarten Substituenten Y¹ und Y² aus.

Bevorzugt sind Komplexkatalysatoren, die mindestens ein Element ausgewählt aus Ruthenium und Iridium enthalten.

In einer bevorzugten Ausführungsform wird das erfindungsgemäße Verfahren in Gegenwart mindestens eines Komplexkatalysators, der mindestens ein Element ausgewählt aus den Gruppen 8, 9 und 10 des Periodensystems sowie mindestens einen Phosphordonorliganden der allgemeinen Formel (XXI) enthält, durchgeführt, wobei
- n: ist 0 oder 1;
- R²¹, R²², R²³, R²⁴, R²⁵, R²⁶: sind unabhängig voneinander unsubstituiertes C₁-C₁₀-Alkyl, C₃-C₁₀-Cycloalkyl, C₃-C₁₀-Heterocyclyl enthaltend mindestens ein Heteroatom ausgewählt aus N, O und S, C₅-C₁₄-Aryl oder C₅-C₁₀-Heteroaryl enthaltend mindestens ein Heteroatom ausgewählt aus N, O und S;
- A: ist
i) eine Brückengruppe ausgewählt aus der Gruppe unsubstituiertes C₁-C₆-Alkan, C₃-C₁₀-Cycloalkan, C₃-C₁₀-Heterocycloalkan enthaltend mindestens ein Heteroatom ausgewählt aus N, O und S, C₅-C₁₄-Aromat und C₅-C₆-Heteroaromat enthaltend mindestens ein Heteroatom ausgewählt aus N, O und S;
   oder
ii) eine Brückengruppe der Formel (XXII) oder (XXIII): 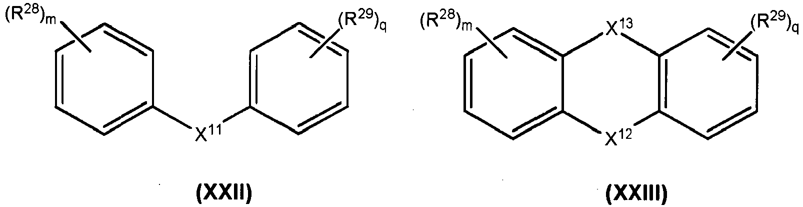
- m, q: sind unabhängig voneinander 0, 1, 2, 3 oder 4;
- R²⁸, R²⁹: sind unabhängig voneinander ausgewählt aus der Gruppe C₁-C₁₀-Alkyl, F, Cl, Br, OH, OR²⁷, NH₂, NHR²⁷ und N(R²⁷)₂,
wobei R²⁷ ausgewählt ist aus C₁-C₁₀-Alkyl und C₅-C₁₀-Aryl;
- X¹¹, X¹²: sind unabhängig voneinander NH, O oder S;
- X¹³: ist eine Bindung, NH, NR³⁰, O, S oder CR³¹R³²;
- R³⁰: ist unsubstituiertes C₁-C₁₀-Alkyl, C₃-C₁₀-Cycloalkyl, C₃-C₁₀-Heterocyclyl enthaltend mindestens ein Heteroatom ausgewählt aus N, O und S, C₅-C₁₄-Aryl oder C₅-C₁₀-Heteroaryl enthaltend mindestens ein Heteroatom ausgewählt aus N, O und S;
- R³¹, R³²: sind unabhängig voneinander unsubstituiertes C₁-C₁₀-Alkyl, C₁-C₁₀-Alkoxy, C₃-C₁₀-Cycloalkyl, C₃-C₁₀-Cycloalkoxy, C₃-C₁₀-Heterocyclyl enthaltend mindestens ein Heteroatom ausgewählt aus N, O und S, C₅-C₁₄-Aryl, C₅-C₁₄-Aryloxy oder C₅-C₁₀-Heteroaryl enthaltend mindestens ein Heteroatom ausgewählt aus N, O und S;
- Y¹, Y², Y³: sind unabhängig voneinander eine Bindung, unsubstituiertes Methylen, Ethylen, Trimethylen, Tetramethylen, Pentamethylen oder Hexamethylen.

In einer weiteren bevorzugten Ausführungsform wird das erfindungsgemäße Verfahren in Gegenwart mindestens eines Komplexkatalysators, der mindestens ein Element ausgewählt aus den Gruppen 8, 9 und 10 des Periodensystems sowie mindestens einen Phosphordonorliganden der allgemeinen Formel (XXV) enthält, 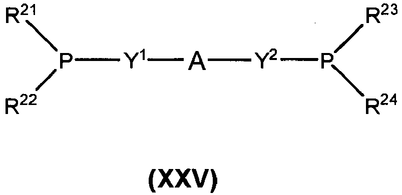 durchgeführt, wobei
- R²¹, R²², R²³, R²⁴: sind unabhängig voneinander unsubstituiertes oder zumindest monosubstituiertes C₁-C₁₀-Alkyl, C₁-C₄-Alkyldiphenylphosphin (-C₁-C₄-Alkyl-P(Phenyl)₂), C₃-C₁₀-Cycloalkyl, C₃-C₁₀-Heterocyclyl enthaltend mindestens ein Heteroatom ausgewählt aus N, O und S, C₅-C₁₄-Aryl oder C₅-C₁₀-Heteroaryl enthaltend mindestens ein Heteroatom ausgewählt aus N, O und S,
wobei die Substituenten ausgewählt sind aus der Gruppe bestehend aus: F, Cl, Br, OH, CN, NH₂ und C₁-C₁₀-Alkyl;
- A: ist
i) eine Brückengruppe ausgewählt aus der Gruppe unsubstituiertes oder zumindest monosubstituiertes N, O, P, C₁-C₆-Alkan, C₃-C₁₀-Cycloalkan, C₃-C₁₀-Heterocycloalkan enthaltend mindestens ein Heteroatom ausgewählt aus N, O und S, C₅-C₁₄-Aromat und C₅-C₆-Heteroaromat enthaltend mindestens ein Heteroatom ausgewählt aus N, O und S,
   wobei die Substituenten ausgewählt sind aus der Gruppe bestehend aus: C₁-C₄-Alkyl, Phenyl, F, Cl, Br, OH, OR²⁷, NH₂, NHR²⁷ oder N(R²⁷)₂,
   wobei R²⁷ ausgewählt ist aus C₁-C₁₀-Alkyl und C₅-C₁₀-Aryl;
   oder
ii) eine Brückengruppe der Formel (XXII) oder (XXIII): 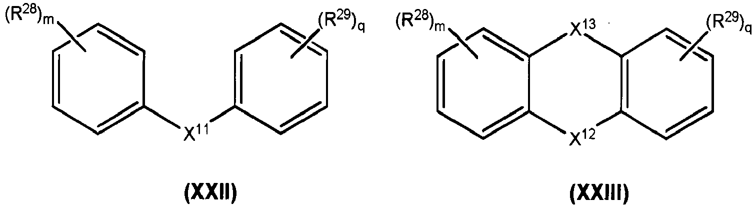
- m, q: sind unabhängig voneinander 0, 1, 2, 3 oder 4;
- R²⁸, R²⁹: sind unabhängig voneinander ausgewählt aus der Gruppe C₁-C₁₀-Alkyl, F, Cl, Br, OH, OR²⁷, NH₂, NHR²⁷ und N(R²⁷)₂,
wobei R²⁷ ausgewählt ist aus C₁-C₁₀-Alkyl und C₅-C₁₀-Aryl;
- X¹¹, X¹²: sind unabhängig voneinander NH, O oder S,
- X¹³: ist eine Bindung, NH, NR³⁰, O, S oder CR³¹ R³²;
- R³⁰: ist unsubstituiertes oder zumindest monosubstituiertes C₁-C₁₀-Alkyl, C₃-C₁₀-Cycloalkyl, C₃-C₁₀-Heterocyclyl enthaltend mindestens ein Heteroatom ausgewählt aus N, O und S, C₅-C₁₄-Aryl oder C₅-C₁₀-Heteroaryl enthaltend mindestens ein Heteroatom ausgewählt aus N, O und S,
wobei die Substituenten ausgewählt sind aus der Gruppe bestehend aus: F, Cl, Br, OH, CN, NH₂ und C₁-C₁₀-Alkyl;
- R³¹, R³²: sind unabhängig voneinander unsubstituiertes oder zumindest monosubstituiertes C₁-C₁₀-Alkyl, C₁-C₁₀-Alkoxy, C₃-C₁₀-Cycloalkyl, C₃-C₁₀-Cycloalkoxy,C₃-C₁₀-Heterocyclyl enthaltend mindestens ein Heteroatom ausgewählt aus N, O und S, C₅-C₁₄-Aryl, C₅-C₁₄-Aryloxy oder C₅-C₁₀-Heteroaryl enthaltend mindestens ein Heteroatom ausgewählt aus N, O und S,
wobei die Substituenten ausgewählt sind aus der Gruppe bestehend aus: F, Cl, Br, OH, CN, NH₂ und C₁-C₁₀-Alkyl;
- Y¹, Y²: sind unabhängig voneinander eine Bindung, unsubstituiertes oder zumindest monosubstituiertes Methylen, Ethylen, Trimethylen, Tetramethylen, Pentamethylen oder Hexamethylen,
wobei die Substituenten ausgewählt sind aus der Gruppe bestehend aus: F, Cl, Br, OH, OR²⁷, CN, NH₂, NHR²⁷, N(R²⁷)₂ und C₁-C₁₀-Alkyl,
wobei R²⁷ ausgewählt ist aus C₁-C₁₀-Alkyl und C₅-C₁₀-Aryl.

In einer weiteren bevorzugten Ausführungsform wird das erfindungsgemäße Verfahren in Gegenwart mindestens eines Komplexkatalysators, der mindestens ein Element ausgewählt aus den Gruppen 8, 9 und 10 des Periodensystems sowie mindestens einen Phosphordonorliganden der allgemeinen Formel (XXVI) enthält, 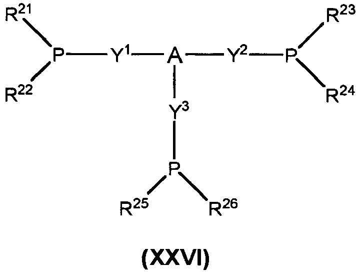 durchgeführt, wobei
- R²¹, R²², R²³, R²⁴, R²⁵, R²⁶: sind unabhängig voneinander unsubstituiertes oder zumindest monosubstituiertes C₁-C₁₀-Alkyl, C₁-C₄-Alkyldiphenylphosphin, C₃-C₁₀-Cycloalkyl, C₃-C₁₀-eterocyclyl enthaltend mindestens ein Heteroatom ausgewählt aus N, O und S, C₅-C₁₄-Aryl oder C₅-C₁₀-Heteroaryl enthaltend mindestens ein Heteroatom ausgewählt aus N, O und S,
wobei die Substituenten ausgewählt sind aus der Gruppe bestehend aus: F, Cl, Br, OH, CN, NH₂ und C₁-C₁₀-Alkyl;
- A: ist eine Brückengruppe ausgewählt aus der Gruppe unsubstituierte oder zumindest monosubstituiertes N, P, C₁-C₆-Alkan, C₃-C₁₀-Cycloalkan, C₃-C₁₀-Heterocycloalkan enthaltend mindestens ein Heteroatom ausgewählt aus N, O und S, C₅-C₁₄-Aromat und C₅-C₆-Heteroaromat enthaltend mindestens ein Heteroatom ausgewählt aus N, O und S,
wobei die Substituenten ausgewählt sind aus der Gruppe bestehend aus: C₁-C₄-Alkyl, Phenyl, F, Cl, Br, OH, OR²⁷, NH₂, NHR²⁷ oder N(R²⁷)₂,
wobei R²⁷ ausgewählt ist aus C₁-C₁₀-Alkyl und C₅-C₁₀-Aryl;
- Y¹, Y², Y³: sind unabhängig voneinander eine Bindung, unsubstituiertes oder zumindest monosubstituiertes Methylen, Ethylen, Trimethylen, Tetramethylen, Pentamethylen oder Hexamethylen,
wobei die Substituenten ausgewählt sind aus der Gruppe bestehend aus: F, Cl, Br, OH, OR²⁷, CN, NH₂, NHR²⁷, N(R²⁷)₂ und C₁-C₁₀-Alkyl,
wobei R²⁷ ausgewählt ist aus C₁-C₁₀-Alkyl und C₅-C₁₀-Aryl.

In einer weiteren bevorzugten Ausführungsform wird das erfindungsgemäße Verfahren in Gegenwart mindestens eines Komplexkatalysators, der mindestens ein Element ausgewählt aus den Gruppen 8, 9 und 10 des Periodensystems sowie mindestens einen beiPhosphordonorliganden der allgemeinen Formel (XXV) enthält, durchgeführt, wobei
- R²¹, R²², R²³, R²⁴: sind unabhängig voneinander Methyl, Ethyl, Isopropyl, tert.-Butyl, Cyclopentyl, Cyclohexyl, Phenyl, oder Mesityl;
- A: ist
i) eine Brückengruppe ausgewählt aus der Gruppe Methan Ethan, Propan, Butan, Cyclohexan, Benzol, Napthalin und Anthracen;
   oder
ii) eine Brückengruppe der Formel (XXVII) oder (XXVIII): 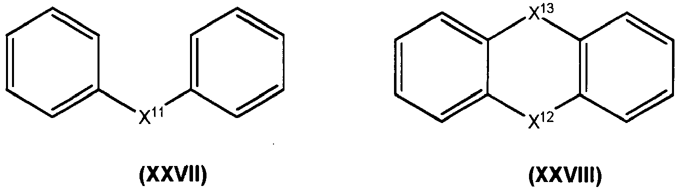
- X¹¹, X¹²: sind unabhängig voneinander NH, O oder S;
- X¹³: ist eine Bindung, NH, O, S oder CR³¹R³²;
- R³¹, R³²: sind unabhängig voneinander unsubstituiertes C₁-C₁₀-Alkyl;
- Y¹, Y²: sind unabhängig voneinander eine Bindung Methylen oder Ethylen.

In einer besonders bevorzugten Ausführungsform wird das erfindungsgemäße Verfahren in Gegenwart mindestens eines Komplexkatalysators, der mindestens ein Element ausgewählt aus den Gruppen 8, 9 und 10 des Periodensystems sowie mindestens einen Phosphordonorliganden der allgemeinen Formel (XXIX) oder (XXX) enthält, 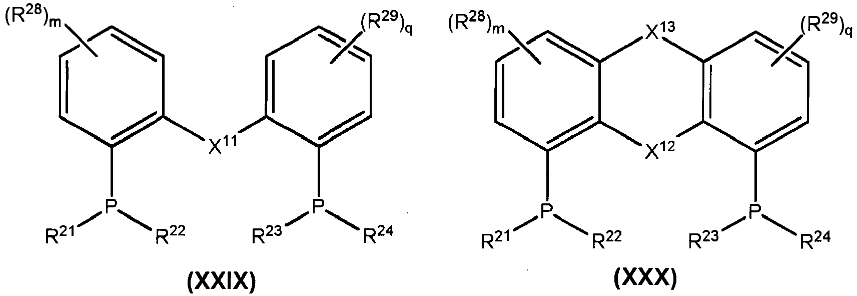 durchgeführt, wobei für m, q, R²¹, R²², R²³, R²⁴, R²⁸, R²⁹, X¹⁹, X¹² und X¹³ die vorstehend aufgeführten Definitionen und Bevorzugungen gelten.

In einer weiteren besonders bevorzugten Ausführungsform wird das erfindungsgemäße Verfahren in Gegenwart mindestens eines Komplexkatalysators, der mindestens ein Element ausgewählt aus der Gruppe Ruthenium und Iridium sowie mindestens einen Phosphordonorliganden ausgewählt aus der Gruppe 1,2-Bis(diphenylphosphino)ethan (dppe), 1,3-Bis(diphenylphosphino)propan (dppp), 1,4-Bis(diphenylphosphino)butan (dppb), 2,3-Bis(dicyclohexylphosphino)ethan (dcpe), 4,5-Bis(diphenylphosphino)-9,9-dimethylxanthen (Xantphos), 1,1,1-Tris(diethylphosphino-methyl)ethan (Rhodaphos), Bis(2-diphenylphosphinoethyl)phenylphosphin und 1,1,1-Tris(Diphenylphosphinomethyl)ethan (Triphos) enthält, durchgeführt.

In einer weiteren besonders bevorzugten Ausführungsform wird das erfindungsgemäße Verfahren in Gegenwart eines Komplexkatalysators, der Ruthenium sowie mindestens einen Phosphordonorliganden ausgewählt aus der Gruppe 4,5-Bis(diphenylphosphino)-9,9-dimethylxanthen (Xantphos), Bis(2-&diphenylphosphinoethyl)phenylphosphin und 1,1,1-Tris(Diphenylphosphinomethyl)ethan (Triphos) enthält, durchgeführt.

In einer weiteren besonders bevorzugten Ausführungsform wird das erfindungsgemäße Verfahren in Gegenwart eines Komplexkatalysators, der Iridium sowie mindestens einen Phosphordonorliganden ausgewählt aus der Gruppe 4,5-Bis(diphenylphosphino)-9,9-dimethylxanthen (Xantphos), Bis(2-diphenylphosphinoethyl)phenylphosphin und 1,1,1-Tris(Diphenylphosphinomethyl)ethan (Triphos) enthält, durchgeführt.

Im Rahmen der vorliegenden Erfindung werden unter C₁-C₁₀-Alkyl verzweigte, unverzweigte, gesättigte und ungesättigte Gruppen verstanden. Bevorzugt sind Alkylgruppen mit 1 bis 6 Kohlenstoffatomen (C₁-C₆-Alkyl). Mehr bevorzugt sind Alkylgruppen mit 1 bis 4 Kohlenstoffatomen (C₁-C₄-Alkyl).

Beispiele für gesättigte Alkylgruppen sind Methyl, Ethyl, n-Propyl, Isopropyl, n-Butyl, Isobutyl, sec.-Butyl, tert.-Butyl, Amyl und Hexyl.

Beispiele für ungesättigte Alkylgruppen (Alkenyl, Alkinyl) sind Vinyl, Allyl, Butenyl, Ethinyl und Propinyl.

Die C₁-C₁₀-Alkylgruppe kann unsubstituiert oder, mit einem oder mehreren Substituenten ausgewählt aus der Gruppe F, Cl, Br, Hydroxy (OH), C₁-C₁₀-Alkoxy, C₅-C₁₀-Aryloxy, C₅-C₁₀-Alkylaryloxy, C₅-C₁₀-Heteroaryloxy enthaltend mindestens ein Heteroatom ausgewählt aus N, O, S, Oxo, C₃-C₁₀-Cycloalkyl, Phenyl, C₅-C₁₀-Heteroaryl enthaltend mindestens ein Heteroatom ausgewählt aus N, O, S, C₅-C₁₀-Heterocyclyl enthaltend mindestens ein Heteroatom ausgewählt aus N, O, S, Naphthyl, Amino, C₁-C₁₀-Alkylamino, C₅-C₁₀-Arylamino, C₅-C₁₀-Heteroarylamino enthaltend mindestens ein Heteroatom ausgewählt aus N, O, S, C₁-C₁₀-Dialkylamino, C₁₀-C₁₂-Diarylamino, C₁₀-C₂₀Alkylarylamino, C₁-C₁₀-Acyl, C₁-C₁₀-Acyloxy, NO₂, C₁-C₁₀-Carboxy, Carbamoyl, Carboxamid, Cyano, Sulfonyl, Sulfonylamino, Sulfinyl, Sulfinylamino, Thiol, C₁-C₁₀-Alkylthiol, C₅-C₁₀-Arylthiol oder C₁-C₁₀-Alkylsulfonyl substituiert sein.
Die vorstehende Definition für C₁-C₁₀-Alkyl gilt für C₁-C₃₀-Alkyl und für C₁-C₆-Alkan sinngemäß.

Unter C₃-C₁₀-Cycloalkyl werden vorliegend gesättigte, ungesättigte monozyklische und polyzyklische Gruppen verstanden. Beispiele für C₃-C₁₀-Cycloalkyl sind Cyclopropyl, Cyclobutyl, Cyclopentyl, Cyclohexyl oder Cycloheptyl. Die Cycloalkylgruppen können unsubstituiert oder substituiert sein mit einem oder mehreren Substituenten, wie sie vorstehend zu der Gruppe C₁-C₁₀-Alkyl definiert wurde.

Die vorstehend genannte Definition für C₃-C₁₀-Cycloalkyl gilt für C₃-C₁₀-Cycloalkan sinngemäß.

### Alkoholaminierung

Die homogenen Katalysatoren können sowohl direkt in ihrer aktiven Form als auch ausgehend von üblichen Vorstufen unter Zugabe der entsprechenden Liganden erst unter den Reaktionsbedingungen erzeugt werden. Übliche Vorstufen sind beispielsweise [Ru(p-cymene)Cl₂]₂, [Ru(benzol)Cl₂]ₙ, [Ru(CO)₂Cl₂]ₙ, [Ru(CO)₃Cl₂]₂ [Ru(COD)(allyl)], [RuCl₃*H₂O], [Ru(acetylacetonat)₃], [Ru(DMSO)₄Cl₂], [Ru(PPh₃)₃(CO)(H)Cl], [Ru(PPh₃)₃(CO)Cl₂], [Ru(PPh₃)₃(CO)(H)₂], [Ru(PPh₃)₃Cl₂], [Ru(cyclopenta-dienyl)(PPh₃)₂Cl], [Ru(cyclopentadienyl)(CO)₂Cl], [Ru(cyclopentadienyl)(CO)₂H], [Ru(cyclopentadienyl)(CO)₂]₂, [Ru(pentamethylcyclopentadienyl)(CO)₂Cl], [Ru(pentamethylcylcopentadienyl)(CO)₂H], [Ru(pentamethylcyclopentadienyl)(CO)₂]₂, [Ru(indenyl)(CO)₂Cl], [Ru(indenyl)(CO)₂H], [Ru(indenyl)(CO)₂]₂, Ruthenocen, [Ru(binap)Cl₂], [Ru(bipyridin)₂Cl₂*2H₂O], [Ru(COD)C)₂]₂, [Ru(pentamethylcyclopentadienyl)(COD)Cl], [Ru₃(CO)₁₂], [Ru(tetraphenylhydroxy-cyclopentadienyl)(CO)₂H], [Ru(PMe₃)₄(H)₂], [Ru(PEt₃)₄(H)₂], [Ru(P*n*Pr₃)₄(H)₂], [Ru(P*n*Bu₃)₄(H)₂], [Ru(P*n*Octyl₃)₄(H)₂], [IrCl₃*H₂O], KIrCl₄, K₃IrCl₆, [Ir(COD)Cl]₂, [Ir(cycloocten)₂Cl]₂, [Ir(ethen)₂Cl]₂, [Ir(cyclopentadienyl)Cl₂]₂, [Ir(pentamethylcyclopentadienyl)Cl₂]₂, [Ir(cylopentadienyl)(CO)₂], [Ir(pentamethylcyclopentadienyl)(CO)₂], [Ir(PPh₃)₂(CO)(H)], [Ir(PPh₃)₂(CO)(Cl)], [Ir(PPh₃)₃(Cl)].

Unter homogen-katalysiert wird im Rahmen der vorliegenden Erfindung verstanden, dass der katalytisch-aktive Teil des Komplexkatalysators zumindest teilweise im flüssigen Reaktionsmedium gelöst vorliegt. In einer bevorzugten Ausführungsform liegen mindestens 90 % des im Verfahren eingesetzten Komplexkatalysators im flüssigen Reaktionsmedium gelöst vor, mehr bevorzugt mindestens 95 %, insbesondere bevorzugt mehr als 99 %, am meisten bevorzugt liegt der Komplexkatalysator vollständig gelöst in flüssigen Reaktionsmedium vor (100%), jeweils bezogen auf die Gesamtmenge im flüssigen Reaktionsmedium.

Die Menge der Metallkomponente des Katalysators, bevorzugt Ruthenium oder Iridium, beträgt im Allgemeinen 0,1 bis 5000 Gewichts-ppm, jeweils bezogen auf das gesamte flüssige Reaktionsmedium.

Die Reaktion erfolgt in der Flüssigphase im Allgemeinen bei einer Temperatur von 20 bis 250°C. Bevorzugt wird das erfindungsgemäße Verfahren bei Temperaturen im Bereich von 100 °C bis 200 °C, besonders bevorzugt im Bereich von 110 bis 160°C durchgeführt.

Die Reaktion kann im Allgemeinen bei einem Gesamtdruck von 0,1 bis 20 MPa absolut, der sowohl der Eigendruck des Lösungsmittels bei der Reaktionstemperatur als auch der Druck eines Gases wie Stickstoff, Argon oder Wasserstoff sein kann, durchgeführt werden. Bevorzugt wird das erfindungsgemäße Verfahren bei einem Gesamtdruck im Bereich von 0,2 bis 15 MPa absolut, insbesondere bevorzugt bei einem Gesamtdruck im Bereich von 1 bis 15 MPa absolut durchgeführt.

Die mittlere Reaktionszeit beträgt im Allgemeinen 15 Minuten bis 100 Stunden.

Das Aminierungsmittel (Ammoniak) kann bezüglich der zu aminierenden Hydroxylgruppen in stöchiometrischen, unterstöchiometrischen oder überstöchiometrischen Mengen eingesetzt werden.

In einer bevorzugten Ausführungsform wird Ammoniak mit einem 1 bis 250-fachen, bevorzugt mit einem 1- bis 100-fachen, insbesondere mit einem 1,5- bis 10-fachen molaren Überschuss pro Mol im Edukt umzusetzender Hydroxylgruppen eingesetzt. Es sind auch höhere Überschüsse an Ammoniak möglich. Der Ammoniak kann gasförmig, flüssig sowie im Lösungsmittel oder Edukt gelöst zugegeben werden.

Das erfindungsgemäße Verfahren kann sowohl in einem Lösungsmittel als auch ohne Lösungsmittel durchgeführt werden. Als Lösungsmittel eignen sich polare und unpolare Lösungsmittel, die in Reinform oder in Mischungen eingesetzt werden können. Beispielsweise kann im erfindungsgemäßen Verfahren nur ein unpolares oder ein polares Lösungsmittel eingesetzt werden. Es ist auch möglich, Mischungen von zwei oder mehr polaren Lösungsmitteln oder Mischungen von zwei oder mehr unpolaren Lösungsmitteln oder Mischungen aus einem oder mehr polaren mit einem oder mehr unpolaren Lösungsmitteln einzusetzen. Auch das Produkt kann als Lösungsmittel in Reinform oder in Mischungen mit polaren oder mit unpolaren Lösungsmittel eingesetzt werden.

Als unpolare Lösungsmittel eignen sich beispielsweise gesättigte und ungesättigte Kohlenwasserstoffe wie Hexan, Heptan, Oktan, Cyclohexan, Benzol, Toluol, Xylol und Mesitylen, und lineare und zyklische Ether wie THF, Diethylether, 1,4-Dioxan, MTBE (tert-Butylmethylether), Diglyme und 1,2-Dimethoxyethan. Bevorzugt werden Toluol, Xylole oder Mesitylen eingesetzt.

Als polare Lösungsmittel eignen sich beispielsweise Wasser, Dimethylformamid, Formamid, tert-Amylalkohol und Acetonitril. Bevorzugt wird Wasser eingesetzt. Das Wasser kann sowohl vor der Reaktion zugesetzt werden, bei der Reaktion als Reaktionswasser entstehen oder auch nach der Reaktion zusätzlich zum Reaktionswasser zugesetzt werden. Ein weiteres bevorzugtes Lösungsmittel ist tert-Amylalkohol.

Für die Umsetzung in der Flüssigphase leitet man Ammoniak, das Diol gegebenenfalls zusammen mit einem oder mehreren Lösungsmitteln, zusammen mit dem Komplexkatalysator in einen Reaktor.
Die Zuleitung von Ammoniak, Diol, gegebenenfalls Lösungsmittel und Komplexkatalysator kann dabei simultan oder getrennt voneinander erfolgen. Die Reaktion kann dabei kontinuierlich, in semibatch-Fahrweise, in batch-Fahrweise, rückvermischt in Produkt als Lösungsmittel oder im geraden Durchgang nicht rückvermischt durchgeführt werden.

Für das erfindungsgemäße Verfahren können prinzipiell alle Reaktoren eingesetzt werden, welche für gas/flüssig-Reaktionen unter der gegebenen Temperatur und dem gegebenen Druck grundsätzlich geeignet sind. Geeignete Standardreaktoren für gas/flüssig- und für flüssig/flüssig-Reaktionssyteme sind beispielsweise in K.D. Henkel, "Reactor Types and Their Industrial Applications", in Ullmann's Encyclopedia of Industrial Chemistry, 2005, Wiley-VCH Verlag GmbH & Co. KGaA, DOI: 10.1002/14356007.b04_087, Kapitel 3.3 "Reactors for gas-liquid reactions" angegeben. Als Beispiele seien genannt Rührkesselreaktoren, Rohrreaktoren oder Blasensäulenreaktoren.

Bei der Aminierungsreaktion wird eine Hydroxylgruppe, bevorzugt eine primäre Hydroxylgruppe (-CH₂-OH) des Edukts mit Ammoniak zu einer primären Aminogruppe (-NH₂) umgesetzt, wobei sich jeweils ein Mol Reaktionswasser pro Mol umgesetzter Hydroxylgruppe bildet.

Die Umsetzung von 1,2-Ethylenglykol führt beispielsweise zum entsprechenden 2-Aminoethanol.

Der bei der Umsetzung entstehende Reaktionsaustrag enthält im Allgemeinen die entsprechenden Alkanolamine, gegebenenfalls das eine oder die mehreren Lösungsmittel, den Komplexkatalysator, gegebenenfalls nicht umgesetzte Edukte und Ammoniak sowie das entstandene Reaktionswasser.

Aus dem Reaktionsaustrag werden der gegebenenfalls vorhandene überschüssige Ammoniak, das gegebenenfalls vorhandene Lösungsmittel, der Komplexkatalysator und das Reaktionswasser entfernt. Das erhaltene Aminierungsprodukt kann weiter aufgearbeitet werden. Der überschlüssige Ammoniak, der Komplexkatalysator, gegebenenfalls das oder die Lösungsmittel und gegebenenfalls nicht umgesetzte Edukte können in die Aminierungsreaktion zurückgeführt werden.

Wird die Aminierungsreaktion ohne Lösungsmittel durchgeführt, so ist der homogene Komplexkatalysator nach der Reaktion im Produkt gelöst. Dieser kann im Produkt verbleiben oder durch eine geeignete Methode aus diesem abgetrennt werden. Möglichkeiten zur Abtrennung des Katalysators sind zum Beispiel das Auswaschen mit einem nicht mit dem Produkt mischbaren Lösungsmittel, in welchem sich der Katalysator durch geeignete Wahl der Liganden besser löst als im Produkt. Gegebenenfalls wird der Katalysator durch mehrstufige Extraktion aus dem Produkt abgereichert. Als Extraktionsmittel wird bevorzugt ein auch für die Zielreaktion geeignetes Lösungsmittel wie Toluol, Benzol, Xylole, Alkane, wie Hexane, Heptane und Oktane, und azyklische oder zyklische Ether, wie Diethylether und Tetrahydrofuran eingesetzt, welches nach dem Einengen zusammen mit dem extrahierten Katalysator wieder für die Umsetzung eingesetzt werden kann. Möglich ist auch die Entfernung des Katalysators mit einem geeigneten Absorbermaterial. Eine Abtrennung kann auch durch Zufügen von Wasser zu der Produktphase erfolgen, falls die Reaktion in einem mit Wasser nicht mischbaren Lösungsmittel durchgeführt wird. Löst sich der Katalysator dabei bevorzugt in dem Lösungsmittel, kann er mit dem Lösungsmittel von der wässrigen Produktphase abgetrennt und gegebenenfalls erneut eingesetzt werden. Dies kann durch Wahl geeigneter Liganden bewirkt werden. Die resultierenden wässrigen Di-, Tri-, oder Polyamine können direkt als technische Aminlösungen eingesetzt werden. Es ist auch möglich, das Aminierungsprodukt durch Destillation vom Katalysator zu trennen.

Wird die Reaktion in einem Lösungsmittels durchgeführt, so kann dieses mit dem Aminierungsprodukt mischbar sein und nach der Reaktion durch Destillation abgetrennt werden. Es können auch Lösungsmittel eingesetzt werden, welche eine Mischungslücke mit den Aminierungsprodukten oder den Edukten aufweisen. Als geeignete Lösungsmittel hierfür seien beispielsweise Toluol, Benzol, Xylole, Alkane, wie Hexane, Heptane und Oktane, und azyklische oder zyklische Ether, wie Diethylether, Tetrahydrofuran und Dioxan genannt. Durch geeignete Wahl der Phosphinliganden löst sich der Katalysator bevorzugt in der Lösungsmittelphase. Die Phosphinliganden können auch so gewählt werden, dass sich der Katalysator im Aminierungsprodukt löst. In diesem Fall lässt sich das Aminierungsprodukt durch Destillation vom Katalysator trennen.

Das Lösungsmittel kann auch unter den Reaktionsbedingungen mit den Edukten und dem Produkt mischbar sein und erst nach dem Abkühlen eine zweite flüssige Phase ausbilden, welche den Großteil des Katalysators enthält. Als Lösungsmittel, die diese Eigenschaft zeigen, seien beispielsweise Toluol, Benzol, Xylole, Alkane, wie Hexane, Heptane und Oktane, genannt. Der Katalysator kann dann zusammen mit dem Lösungsmittel abgetrennt und wieder verwendet werden. Die Produktphase kann auch in dieser Variante mit Wasser versetzt werden. Der in dem Produkt enthaltene Teil des Katalysators kann anschließend durch geeignete Absorbermaterialien wie beispielsweise Polyacrylsäure und deren Salze, sulfonierte Polystyrole und deren Salze, Aktivkohlen, Montmorillonite, Bentonite sowie Zeolithe abgetrennt werden oder aber in dem Produkt belassen werden.

Die Aminierungsreaktion kann auch zweiphasig durchgeführt werden. Bei der Ausführungsform der zweiphasigen Reaktionsführung eignen sich als unpolare Lösungsmittel besonders Toluol, Benzol, Xylole, Alkane, wie Hexane, Heptane und Oktane, in Kombination mit lipophilen Phosphinliganden am Übergangsmetallkatalysator, wodurch sich der Übergangsmetallkatalysator in der unpolaren Phase anreichert. Bei dieser Ausführungsform, in welcher das Produkt sowie das Reaktionswasser und ggf. nicht umgesetzte Edukte eine mit diesen Verbindungen angereicherte Zweitphase bilden, kann der Großteil des Katalysators durch einfache Phasentrennung von der Produktphase abgetrennt und wiederverwendet werden.

Falls flüchtige Nebenprodukte oder nicht umgesetzte Edukte oder auch das bei der Reaktion entstandene oder nach der Reaktion zur besseren Extraktion zugesetzte Wasser unerwünscht sind, können diese problemlos von dem Produkt durch Destillation abgetrennt werden.

Es kann auch vorteilhaft sein, das bei der Reaktion gebildete Wasser kontinuierlich aus dem Reaktionsgemisch zu entfernen. Das Reaktionswasser kann direkt durch Destillation aus dem Reaktionsgemisch oder als Azeotrop unter Zusatz eines geeigneten Lösungsmittels (Schleppers) und unter Verwendung eines Wasserabscheiders abgetrennt, oder durch Zusatz von Wasser entziehenden Hilfsstoffen entfernt werden.

Der Zusatz von Basen kann einen positiven Effekt auf die Produktbildung haben. Als geeignete Basen seien hier Alkalihydroxide, Erdalkalihydroxide, Alkalialkoholate, Erdalkalialkoholate, Alkali-Carbonate und Erdalkalicarbonate genannt, die in Mengen von 0,01 bis 100 molaren Äquivalenten in Bezug auf den verwendeten Metallkatalysator eingesetzt werden können.

Ein weiterer Gegenstand der vorliegenden Erfindung ist die Verwendung eines Komplexkatalysators, der mindestens ein Element ausgewählt aus den Gruppen 8, 9 und 10 des Periodensystems sowie mindestens einen Donorliganden enthält, zur homogen-katalysierten Herstellung von Alkanolaminen, die eine primäre Aminogruppe (-NH₂) und eine Hydroxylgruppe (-OH) aufweisen, durch Alkoholaminierung von Diolen, die zwei Hydroxylgruppen (-OH) aufweisen, mit Ammoniak.

In einer bevorzugten Ausführungsform betrifft die vorliegende Erfindung die Verwendung eines homogen gelösten Komplexkatalysators der allgemeinen Formel (I): 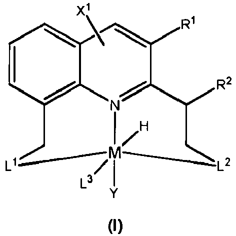 in der
- L¹ und L²: unabhängig voneinander PR^{a}R^{b}, NR^{a}R^{b}, Sulfid, SH, S(=O)R, C₅-C₁₀-Heteroaryl enthaltend mindestens ein Heteroatom ausgewählt aus N, O und S, AsR^{a}R^{b}, SbR^{a}R^{b} oder N-heterozyklische Carbene der Formel II oder III: 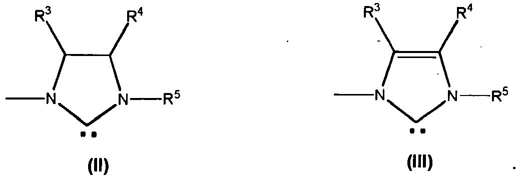
- L³: einzähniger Zweielektronendonor ausgewählt aus der Gruppe CO, PR^{a}R^{b}R^{c}, NO⁺, AsR^{a}R^{b}R^{c}, SbR^{a}R^{b}R^{c}, SR^{a}R^{b}, RCN, RNC, N₂, PF₃, CS, Pyridin, Thiophen, Tetrahydrothiophen und N-heterozyklische Carbene der Formel (II) oder (III);
- R¹ und R²: beide Wasserstoff oder gemeinsam mit den Kohlenstoffatomen, an die sie gebunden sind, ein Phenylring, der zusammen mit der Chinolinyleinheit der Formel (I) eine Acridinyleinheit bildet;
- R, R^{a}, R^{b}, R^{c}, R³, R⁴, und R⁵: unabhängig voneinander unsubstituiertes oder zumindest monosubstituiertes C₁-C₁₀-Alkyl, C₃-C₁₀-Cycloalkyl, C₃-C₁₀-Heterocyclyl enthaltend mindestens ein Heteroatom ausgewählt aus N, O und S, C₅-C₁₀-Aryl oder C₅-C₁₀-Heteroaryl enthaltend mindestens ein Heteroatom ausgewählt aus N, O und S,
wobei die Substituenten ausgewählt sind aus der Gruppe bestehend aus: F, Cl, Br, OH, CN, NH₂ und C₁-C₁₀-Alkyl;
- Y: monoanionischer Ligand ausgewählt aus der Gruppe H, F, Cl, Br, I, OCOR, OCOCF₃, OSO₂R, OSO₂CF₃, CN, OH, OR und N(R)₂ oder neutrale Moleküle ausgewählt aus der Gruppe NH₃, N(R)₃ und R₂NSO₂R;
- X¹: ein, zwei, drei, vier, fünf, sechs, oder sieben Substituenten an einem oder mehreren Atomen der Acridinyleinheit oder ein, zwei, drei, vier oder fünf Substituenten an einem oder mehreren Atomen der Chinolinyleinheit,
wobei X¹ unabhängig voneinander ausgewählt ist aus der Gruppe bestehend aus Wasserstoff, F, Cl, Br, I, OH, NH₂, NO₂, -NC(O)R, C(O)NR₂, -OC(O)R, -C(O)OR, CN und Boranderivaten, die aus dem Katalysator der Formel I durch Reaktion mit NaBH₄ erhältlich sind und unsubstituiertem oder zumindest monosubstituiertem C₁-C₁₀-Alkoxy, C₁-C₁₀-Alkyl, C₃-C₁₀-Cycloalkyl, C₃-C₁₀-Heterocyclyl enthaltend mindestens ein Heteroatom ausgewählt aus N, O und S, C₅-C₁₀-Aryl und C₅-C₁₀-Heteroaryl enthaltend mindestens ein Heteroatom ausgewählt aus N, O und S,
wobei die Substituenten ausgewählt sind aus der Gruppe bestehend aus: F, Cl, Br, OH, CN, NH₂ und C₁-C₁₀-Alkyl;
und
- M: Eisen, Cobalt, Nickel, Ruthenium, Rhodium, Palladium, Osmium, Iridium oder Platin
bedeuten, zur homogen-katalysierten Herstellung von Alkanolaminen, die eine primäre Aminogruppe (-NH₂) und eine Hydroxylgruppe (-OH) aufweisen, durch Alkoholaminierung von Diolen, die zwei Hydroxygruppen (-OH) aufweisen, mit Ammoniak, wobei für den Katalysator der allgemeinen Formel (I) die vorstehend für das erfindungsgemäße Verfahren beschriebenen Definitionen und Bevorzugungen gelten.

In einer weiteren bevorzugten Ausführungsform betrifft die vorliegende Erfindung die Verwendung eines homogen gelösten Komplexkatalysators der allgemeinen Formel (XV): 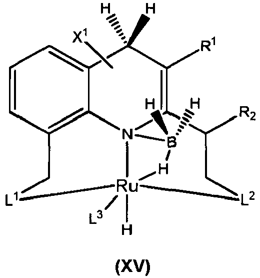 in der
- L¹ und L²: unabhängig voneinander PR^{a}R^{b}, NR^{a}R^{b}, Sulfid, SH, S(=O)R, C₅-C₁₀-Heteroaryl enthaltend mindestens ein Heteroatom ausgewählt aus N, O und S, AsR^{a}R^{b}, SbR^{a}R^{b} oder N-heterozyklische Carbene der Formel (II) oder (III): 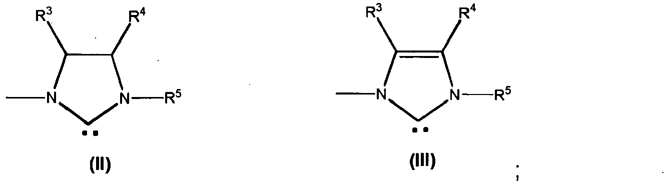
- L³: einzähniger Zweielektronendonor ausgewählt aus der Gruppe CO, PR^{a}R^{b}R^{c}, NO⁺, AsR^{a}R^{b}R^{c}, SbR^{a}R^{b}R^{c}, SR^{a}R^{b}, RCN, RNC, N₂, PF₃, CS, Pyridin, Thiophen, Tetrahydrothiophen und N-heterozyklische Carbene der Formel (II) oder (III);
- R¹ und R²: beide Wasserstoff oder gemeinsam mit den Kohlenstoffatomen, an die sie gebunden sind, ein Phenylring, der zusammen mit der Chinolinyleinheit der Formel (I) eine Acridinyleinheit bildet;
- R, R^{a}, R^{b}, R^{c}, R³, R⁴, und R⁵: unabhängig voneinander unsubstituiertes oder zumindest monosubstituiertes C₁-C₁₀-Alkyl, C₃-C₁₀-Cycloalkyl, C₃-C₁₀-Heterocyclyl enthaltend mindestens ein Heteroatom ausgewählt aus N, O und S, C₅-C₁₀-Aryl oder C₅-C₁₀-Heteroaryl enthaltend mindestens ein Heteroatom ausgewählt aus N, O und S,
wobei die Substituenten ausgewählt sind aus der Gruppe bestehend aus: F, Cl, Br, OH, CN, NH₂ und C₁-C₁₀-Alkyl;
- Y: monoanionischer Ligand ausgewählt aus der Gruppe H, F, Cl, Br, I, OCOR, OCOCF₃, OSO₂R, OSO₂CF₃, CN, OH, OR und N(R)₂ oder neutrale Moleküle ausgewählt aus der Gruppe NH₃, N(R)₃ und R₂NSO₂R;
- X¹: ein, zwei, drei, vier, fünf, sechs, oder sieben Substituenten an einem oder mehreren Atomen der Acridinyleinheit oder ein, zwei, drei, vier oder fünf Substituenten an einem oder mehreren Atomen der Chinolinyleinheit,
wobei X¹ unabhängig voneinander ausgewählt ist aus der Gruppe bestehend aus Wasserstoff, F, Cl, Br, I, OH, NH₂, NO₂, -NC(O)R, C(O)NR₂, -OC(O)R, -C(O)OR, CN und Boranderivaten, die aus dem Katalysator der Formel I durch Reaktion mit NaBH₄ erhältlich sind und unsubstituiertem oder zumindest monosubstituiertem C₁-C₁₀-Alkoxy, C₁-C₁₀-Alkyl, C₃-C₁₀-Cycloalkyl, C₃-C₁₀-Heterocyclyl enthaltend mindestens ein Heteroatom ausgewählt aus N, O und S, C₅-C₁₀-Aryl und C₅-C₁₀-Heteroaryl enthaltend mindestens ein Heteroatom ausgewählt aus N, O und S,
wobei die Substituenten ausgewählt sind aus der Gruppe bestehend aus: F, Cl, Br, OH, CN, NH₂ und C₁-C₁₀-Alkyl;
und
- M: Eisen, Cobalt, Nickel, Ruthenium, Rhodium, Palladium, Osmium, Iridium oder Platin

bedeuten, zur homogen-katalysierten Herstellung von Alkanolaminen, die eine primäre Aminogruppe (-NH₂) und eine Hydroxylgruppe (-OH) aufweisen, durch Alkoholaminierung von Diolen, die zwei Hydroxylgruppen (-OH) aufweisen, mit Ammoniak, wobei für den Katalysator der allgemeinen Formel (XV) die vorstehend für das erfindungsgemäße Verfahren beschriebenen Definitionen und Bevorzugungen gelten.

Ein weiterer Gegenstand der vorliegenden Erfindung ist die Verwendung eines Komplexkatalysators, der mindestens ein Element ausgewählt aus den Gruppen 8, 9 und 10 des Periodensystems sowie mindestens einen Phosphordonorliganden der allgemeine Formel (XXI) enthält, 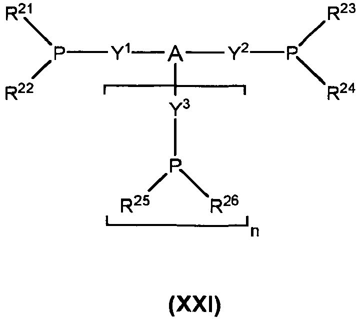 wobei
- n: ist 0 oder 1;
- R²¹, R²², R²³, R²⁴, R²⁵, R²⁶: sind unabhängig voneinander unsubstituiertes oder zumindest monosubstituiertes C₁-C₁₀-Alkyl, C₁-C₄-Alkyldiphenylphosphin (-C₁-C₄-Alkyl-P(Phenyl)₂), C₃-C₁₀-Cycloalkyl, C₃-C₁₀-Heterocyclyl enthaltend mindestens ein Heteroatom ausgewählt aus N, O und S, C₅-C₁₄-Aryl oder C₅-C₁₀-Heteroaryl enthaltend mindestens ein Heteroatom ausgewählt aus N, O und S,
wobei die Substituenten ausgewählt sind aus der Gruppe bestehend aus: F, Cl, Br, OH, CN, NH₂ und C₁-C₁₀-Alkyl;
- A: ist
i) eine Brückengruppe ausgewählt aus der Gruppe unsubstituiertes oder zumindest monosubstituiertes N, O, P, C₁-C₆-Alkan, C₃-C₁₀-Cycloalkan, C₃-C₁₀-Heterocycloalkan enthaltend mindestens ein Heteroatom ausgewählt aus N, O und S, C₅-C₁₄-Aromat oder C₅-C₆-Heteroaromat enthaltend mindestens ein Heteroatom ausgewählt aus N, O und S,
   wobei die Substituenten ausgewählt sind aus der Gruppe bestehend aus: C₁-C₄-Alkyl, Phenyl, F, Cl, Br, OH, OR²⁷, NH₂, NHR²⁷ oder N(R²⁷)₂,
   wobei R²⁷ ausgewählt ist aus C₁-C₁₀-Alkyl und C₅-C₁₀-Aryl;
   oder
ii) eine Brückengruppe der Formel (XXII) oder (XXIII): 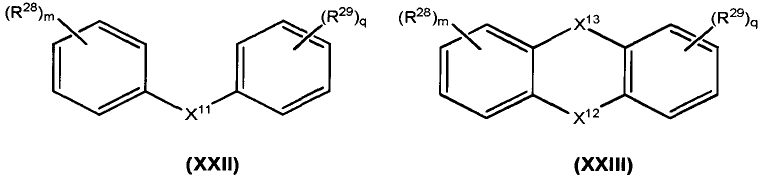
- m, q: sind unabhängig voneinander 0, 1, 2, 3 oder 4;
- R²⁸, R²⁹: sind unabhängig voneinander ausgewählt aus der Gruppe C₁-C₁₀-Alkyl, F, Cl, Br, OH, OR²⁷, NH₂, NHR²⁷ und N(R²⁷)₂, wobei R²⁷ ausgewählt ist aus C₁-C₁₀-Alkyl und C₅-C₁₀-Aryl;
- X¹¹, X¹²: sind unabhängig voneinander NH, O oder S;
- X¹³: ist eine Bindung, NH, NR³⁰, O, S oder CR³¹R³²;
- R³⁰: ist unsubstituiertes oder zumindest monosubstituiertes C₁-C₁₀-Alkyl, C₃-C₁₀-Cycloalkyl, C₃-C₁₀-Heterocyclyl enthaltend mindestens ein Heteroatom ausgewählt aus N, O und S, C₅-C₁₄-Aryl oder C₅-C₁₀-Heteroaryl enthaltend mindestens ein Heteroatom ausgewählt aus N, O und S, wobei die Substituenten ausgewählt sind aus der Gruppe bestehend aus: F, Cl, Br, OH, CN, NH₂ und C₁-C₁₀-Alkyl;
- R³¹, R³²: sind unabhängig voneinander unsubstituiertes oder zumindest monosubstituiertes C₁-C₁₀-Alkyl, C₁-C₁₀-Alkoxy, C₃-C₁₀-Cycloalkyl, C₃-C₁₀-Cycloalkoxy,C₃-C₁₀-Heterocyclyl enthaltend mindestens ein Heteroatom ausgewählt aus N, O und S, C₅-C₁₄-Aryl, C₅-C₁₄-Aryloxy oder C₅-C₁₀-Heteroaryl enthaltend mindestens ein Heteroatom ausgewählt aus N, O und S, wobei die Substituenten ausgewählt sind aus der Gruppe bestehend aus: F, Cl, Br, OH, CN, NH₂ und C₁-C₁₀-Alkyl;
- Y¹, Y², Y³: sind unabhängig voneinander eine Bindung, unsubstituiertes oder zumindest monosubstituiertes Methylen, Ethylen, Trimethylen, Tetramethylen, Pentamethylen oder Hexamethylen, wobei die Substituenten ausgewählt sind aus der Gruppe bestehend aus: F, Cl, Br, OH, OR²⁷, CN, NH₂, NHR²⁷, N(R²⁷)₂ und C₁-C₁₀-Alkyl, wobei R²⁷ ausgewählt ist aus C₁-C₁₀-Alkyl und C₅-C₁₀-Aryl,
zur homogen-katalysierten Herstellung von Alkanolaminen, die eine primäre Aminogruppe und eine Hydroxylgruppe aufweisen, durch Alkoholaminierung von Diolen, die zwei Hydroxylgruppe (-OH) aufweisen, mit Ammoniak.

Für die Verwendung des Komplexkatalysators der Formel (XXI) zur homogen-katalysierten Herstellung von Alkanolaminen, die eine primäre Aminogruppe (-NH₂) und eine Hydroxylgruppe (-OH) aufweisen, durch Alkoholaminierung von Diolen, die zwei Hydroxylgruppen (-OH) aufweisen, mit Ammoniak gelten die für das erfindungsgemäße Verfahren beschriebenen Definitionen und Bevorzugungen.

Die Erfindung wird durch die nachfolgenden Beispiele verdeutlicht, ohne sie darauf zu beschränken.

### Beispiel

### Generelle Vorschrift für die erfindungsgemäße katalytische Aminierung von Alkoholen mit Ammoniak

Ligand L, Metallsalz M oder Katalysatorkomplex XIVb (Herstellung, siehe unten, Einwaage unter interter Atmosphäre), Lösungsmittel und der umzusetzende Alkohol wurden unter Ar-Atmosphäre in einem 160 ml Parr-Autoklaven (Edelstahl V4A) mit magnetisch gekoppeltem Schrägblattrührer (Rührgeschwindigkeit: 200-500 Umdrehungen/Minute) vorgelegt. Die angegebene Menge an Ammoniak wurde bei Raumtemperatur entweder vorkondensiert oder direkt aus der NH₃-Druckgasflasche zudosiert. Falls Wasserstoff eingesetzt wurde, erfolgte dies durch iterative Differenzdruck-Dosierung. Der Stahlautoklav wurde auf die angegebene Temperatur elektrisch aufgeheizt und für die angegebene Zeit unter Rühren (500 Umdrehungen/Minute) erhitzt (Innentemperaturmessung). Nach dem Abkühlen auf Raumtemperatur, Entspannung des Autoklaven und Ausgasen des Ammoniaks bei Normaldruck wurde die Reaktionsmischung mittels GC (30m RTX5 Amin 0,32mm 1,5µm) analysiert. Die Ergebnisse für die Aminierung von 1,4-Butandiol (Tabellen 1a, 1b und 2), Diethylenglykol (Tabellen 3a, 3b und 4), Monoethylenglykol (Tabelle 5),Diethanolamin (Tabelle 6), 1,5-Pentandiol, 1,9-Nonandiol, 1,6-Hexandiol und 1,10-Decandiol (Tabelle 7) sowie 2,5-Dimethanolfuran (Tabelle 8) sind im Folgenden angegeben.

### Synthese des Katalysatorkomplexes XIVb

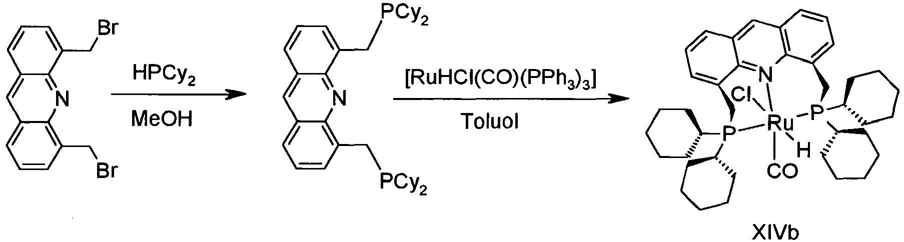

### a) Synthese von 4,5-Bis(dicyclohexylphosphinomethyl)acridin

Eine Lösung aus 4,5-Bis(bromomethyl)acridin¹ (5,2 g, 14,2 mmol) und Dicyclohexylphosphin (8,18 g, 36,8 mmol) in 65 mL wasserfreiem, entgasten Methanol wurde 66h auf 50°C unter einer inerten Argon-Atmosphäre erhitzt. Nach Abkühlen auf Raumtemperatur wurde Triethylamin (5,72 g, 56,7 mmol) zugegeben und 1h gerührt. Verdampfen des Lösungsmittels ergab einen gelb-weißen Feststoff in rotem Öl. Extraktion mittels 3 x 40 mL MTBE und Konzentration des Filtrats ergaben ein rotbraunes Öl (¹H NMR: Mischung aus Produkt & HPCy₂). Aufnehmen in wenig warmen MTBE gefolgt von Zugabe von eisgekühltem Methanol resultierte in Ausfällung eines gelben, mikrokristallinen Feststoffes. Isolierung und Trocknung im Vakuum ergab luftempfindliches 4,5-Bis(dicyclohexylphosphinomethyl)acridin (2,74 g, 33%) als gelbes Pulver. ¹H NMR (360,63 MHz, Toluol-d8): δ [ppm] = 8,07 (s, 1H, H9), 7,91 (d, J = 8,3 Hz, 2H, Ar-H), 7,42 (d, J = 8,3 Hz, 2H, Ar-H), 7,21 (dd, J = 8,3 Hz, J = 7,2 Hz, 2H, Ar-H), 3,89 (bs, 4H, -CH₂-P), 1,96-1,85 (m, 8H, Cy-H), 1,77-1,54 (m, 20H, Cy-H), 1,26-1,07 (m, 16H, Cy-H). ³¹P{¹H} NMR (145,98 MHz, Toluol-d8): δ [ppm] = 2,49 (s, -CH₂-P(Cy)₂).

### b) Synthese des Katalysatorkomplexes XIVb

4,5-Bis(dicyclohexylphosphinomethyl)acridin (1855 mg, 3,1 mmol) und [RuHCl(CO)(PPh₃)₃]² (2678 mg, 2,81 mmol) wurden für 2h in 80 mL entgastem Toluol auf 70°C erhitzt. Die resultierende dunkelbraune Lösung wurde zur Trockene eingeengt, der Rückstand in 3 x 20 mL Hexan aufgeschlämmt und durch Filtration isoliert. Trocknen im Vakuum ergab Ru-PNP-Pincer-Komplex XIVb (1603 mg, 75%) als orange-braunes Pulver. ¹H NMR (360,63 MHz, Toluol-d8): δ [ppm] = 8,06 (s, 1H, H9), 7,43 (d, J = 7,6 Hz, 2H, Ar-H), 7,33 (d, J = 6,5 Hz, 2H, Ar-H), 7,06-7,02 (m, 2H, Ar-H), 5,02 (d, J = 11,9 Hz, 2H, -CHH-PCy₂), 3,54 (d, J = 12,2 Hz, 2H, -CHH-PCy₂), 2,87 (bs, 2H, - P(CₐH(CH₂)₅)₂), 2,54 (bs, 2H, -P(C_{b}H(CH₂)₅)₂), 2,18 (bs, 2H, Cy-H), 1,88-1,85 (m, 8H, Cy-H), 1,65 (bs, 6H, Cy-H), 1,42-1,35 (m, 14H, Cy-H), 1,17-0,82 (m, 12H, Cy-H), - 16,29 (t, J = 19,1 Hz, 1H, Ru-H). ³¹P{¹H} NMR (145,98 MHz, Toluol-d8): δ [ppm] = 60,89 (s, -CH₂-P(Cy)₂).
[1] J. Chiron, J.P. Galy, Synlett, 2003, 15, 2349-2350.
[2] Literaturvorschrift: Inorganic Syntheses 1974, 15, 48. Siehe auch: T. Joseph, S. S. Deshpande, S. B. Halligudi, A. Vinu, S. Ernst, M. Hartmann, J. Mol. Cat. (A) 2003, 206, 13-21.

| **Ligandenbezeichnung** | **CAS** | **IUPAC** |
|---|---|---|
| Triphos | 22031-12-5 | 1,1,1-Tris(Diphenylphosphino-methyl)ethan |
| Xantphos | 161265-03-8 | 4,5-Bis(diphenylphosphino)-9,9-dimethylxanthen |
| Rhodaphos | 22031-14-7 | 1,1,1-Tris(diethylposphino-methyl)ethan |
| DPPEPP | 23582-02-7 | Bis(2-diphenylphosphinoethyl)phenylphosphin |
| Tetraphos | 23582-03-8 | Tris[2-(diphenylphosphino)ethyl]phosphin |
| dppb | 7688-25-7 | 1,4-Bis(diethylphosphino)butan |

**Tabelle 1a: Aminierung von 1,4-Butandiol mit verschiedenen Katalysatorsystemen**

| | | | | | | | | | | | | | |
|---|---|---|---|---|---|---|---|---|---|---|---|---|---|
| 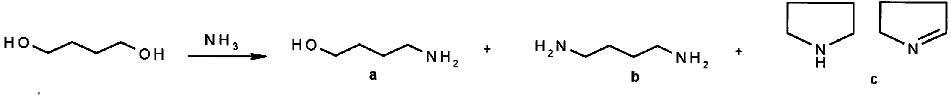 | | | | | | | | | | | | | |

| **Nr** | **Lösungsmittel ^{a)}** | **T [°C]** | **Zeit [h]** | **NH₃ [Eq.]^{e)}** | **Reaktionsdruck [bar]** | **Metallsalz [M]** | **Met. [M] (mol%)^{f)}** | **Ligand [L]** | **Lig. [L] (mol%)^{f)}** | **Umsatz ^{b}** | **Selektiität v^{c}** | | |
|---|---|---|---|---|---|---|---|---|---|---|---|---|---|
| | | | | | | | | | | | **a :** | **b :** | **c** |
| 1 | Toluol | 155 | 12 | 6 | 49 | [RuHCl(CO)(PPh₃)₃] | 0,1 | Triphos | 0,1 | 74,7 | 59,1 | 0,7 | 6,7 |
| 2 | Toluol | 155 | 12 | 6 | 66^{d)} | [RuHCl(CO)(PPh₃)₃] | 0,1 | Triphos | 0,1 | 61,8 | 78,0 | 0,6 | 5,4 |
| 3 | Toluol | 155 | 12 | 6 | 45 | [RuHCl(CO)(PPh₃)₃] | 0,1 | Xantphos | 0,1 | 35,0 | 81,8 | 0,0 | 6,4 |
| 4 | Toluol | 155 | 12 | 6 | 47 | [Ru(COD)methylallyl₂] | 0,1 | Tetraphos | 0,1 | 6,0 | 8,5 | 0,0 | 1,6 |
| 5 | Toluol | 155 | 12 | 6 | 39 | [RuHCl(CO)(PPh₃)₃] | 0,2 | Rhodaphos | 0,2 | 39,8 | 17,5 | 0,0 | 4,6 |
| 6 | Toluol | 155 | 12 | 6 | 38 | [RuHCl(CO)(PPh₃)₃] | 0,2 | DPPEPP | 0,2 | 66,6 | 68,1 | 0,1 | 11,0 |
| a) 50ml Lösungsmittel; Ansatzgröße: 25 mmol 1,4-Butandiol b) Auswertung per GC (FI.%); c) Produktselektivität bestimmt per GC; d) kalt aufgepresst: 5 bar H₂, 8 bar NH₃, e) Molare Equivalente NH₃ pro OH-Funktion am Substrat; f) mol% bezogen auf Anzahl der OH-Funktionen am Substrat; | | | | | | | | | | | | | |

**Tabelle 1b: Aminierung von 1,4-Butandiol mit verschiedenen Katalysatorsystemen**

| **Nr** | **Lösungsmittel ^{a)}** | **T [°C]** | **Zeit [h]** | **NH₃ [Eq.]^{d)}** | **Reaktionsdruck [bar]** | **Metallsalz [M]** | **Met. [M] (mol%)e^{f)}** | **Ligand [L]** | **Lig. [L] (mol%)^{e)}** | **Umsatz ^{b}** | **Selektivität ^{c}** | | |
|---|---|---|---|---|---|---|---|---|---|---|---|---|---|
| | | | | | | | | | | | **a :** | **b :** | **c** |
| 1 | THF | 155 | 12 | 6 | 40 | [RuHCl(CO)(PPh₃)₃] | 0,2 | dppb | 0,2 | 49,6 | 61,4 | 0,0 | 20,3 |
| a) 50ml Lösungsmittel; Ansatzgröße: 25 mmol 1,4-Butandiol b) Auswertung per GC (FI.%); c) Produktselektivität bestimmt per GC; d) Molare Equivalente NH₃ pro OH-Funktion am Substrat; e) mol% bezogen auf Anzahl der OH-Funktionen am Substrat; | | | | | | | | | | | | | |

**Tabelle 2: Aminierung von 1,4-Butandiol mit XIVb als Katalysatorsystem**

| | | | | | | | | | | |
|---|---|---|---|---|---|---|---|---|---|---|
| 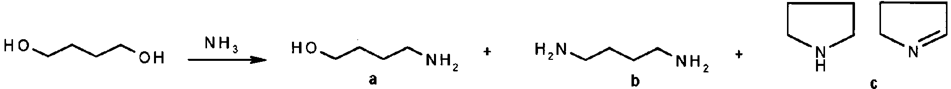 | | | | | | | | | | |

| **Nr^{a)}** | **Lösungsmittel** | **T [°C]** | **Zeit [h]** | **NH₃ [Eq.]^{e)}** | **Reaktionsdruck [bar]** | **Weitere Bedingung** | **Umsatz^{b}** | **Selektivität** | | |
|---|---|---|---|---|---|---|---|---|---|---|
| | | | | | | | | **a** | **b** | **c** |
| 1 | Toluol | 155 | 12 | 6 | 42 | | 54,6 | 72,2 | 8,8 | 17,7 |
| 2 | Toluol | 155 | 12 | 6 | 41 | 5,0 mol% Wasser | 63,0 | 71,8 | 9,3 | 17,3 |
| 3 | Toluol | 155 | 12 | 6 | 55 | 5 bar H₂ kalt aufgepresst | 25,0 | 81,0 | 4,8 | 13,6 |
| 4 | Toluol | 155 | 12 | 9 | 47 | | 61,1 | 74,2 | 8,9 | 15,7 |
| 5 | p-Xylol | 155 | 12 | 6 | 44 | | 70,6 | 62,6 | 5,8 | 28,7 |
| 6 | p-Xylol | 155 | 12 | 6 | 40 | - | 42,0 | 78,8 | 3,5 | 16,5 |
| 7 | p-Xylol | 155 | 12 | 9 | 48 | | 62,3 | 72,3 | 7,6 | 18,5 |
| 8 | p-Xylol | 155 | 12 | 18 | 78 | | 48,1 | 75,9 | 2,6 | 17,4 |
| 9 | Mesitylen * | 155 | 12 | 6 | 39 | - | 58,3 | 70,5 | 6,7 | 20,3 |
| a) Bedingungen wenn nicht anders angegeben: 50 ml Lösungsmittel, Ansatzgröße 25 mmol 1,4-Butandiol, 0.1 mol% Katalysatorkomplex XIVb (bzgl. Anzahl der OH-Funktionen am Substrat), b) Auswertung per GC (FI.%), c) Produktselektivität bestimmt per GC, e) Molare Equivalente NH₃ pro OH-Funktion am Substrat; | | | | | | | | | | |

**Tabelle 3a: Aminierung von Diethylenglykol mit verschiedenen Katalysatorsystemen**

| | | | | | | | | | | | | | |
|---|---|---|---|---|---|---|---|---|---|---|---|---|---|
| 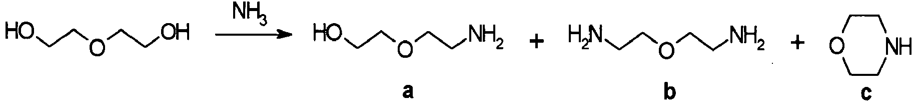 | | | | | | | | | | | | | |

| **Nr^{a)}** | **Lösungsmittel** | **T [°C]** | **Zeit [h]** | **NH₃ [Eq.]^{e)}** | **Reatkionsdruck [bar]** | **Metallsalz [M]** | **Met. [M] (mol%)^{f)}** | **Ligand [L]** | **Lig. [L] (mol%)^{f)}** | **Umsatz ^{b}** | **Selektiivität^{c}** | | |
|---|---|---|---|---|---|---|---|---|---|---|---|---|---|
| | | | | | | | | | | | **a** | **: b** | **: c** |
| 1 | Toluol | 155 | 12 | 6 | 41 | [RuHCl(CO)(PPh₃)₃] | 0,1 | Triphos | 0,1 | 51,0 | 66,2 | 0,9 | 5,9 |
| 2 | Toluol | 155 | 12 | 6 | 59^{d)} | [RuHCl(CO)(PPh₃)₃] | 0,1 | Triphos | 0,1 | 16,2 | 87,3 | 0,1 | 2,3 |
| 3 | Toluol | 180 | 12 | 6 | 43 | [RuHCl(CO)(PPh₃)₃] | 0,2 | Xantphos | 0,1 | 27,7 | 67,1 | 0,2 | 5,3 |
| 4 | Toluol | 155 | 12 | 6 | 44 | [Ru(COD)methylallyl₂] | 0,1 | Tetraphos | 0,1 | 3,9 | 0,0 | 0,0 | 1,1 |
| 5 | Toluol | 155 | 12 | 6 | 40 | [RuHCl(CO)(PPh₃)₃] | 0,2 | Rhodaphos | 0,2 | 21,8 | 4,7 | 0,0 | 1,3 |
| a) 50ml Lösungsmittel; Ansatzgröße: 25 mmol Diethylenglykol b) Auswertung per GC (FI.%); c) Produktselektivität bestimmt per GC; d) kalt aufgepresst: 5 bar H₂, 8 bar NH₃, e) Molare Equivalente NH₃ pro OH-Funktion am Substrat; f) mol% bezogen auf Anzahl der OH-Funktionen am Substrat; | | | | | | | | | | | | | |

**Tabelle 3b: Aminierung von Diethylenglykol mit verschiedenen Katalysatorsystemen**

| | | | | | | | | | | | | | |
|---|---|---|---|---|---|---|---|---|---|---|---|---|---|
| 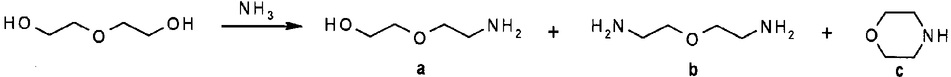 | | | | | | | | | | | | | |

| **Nr^{a)}** | **Lösungsmittel** | **T [°C]** | **Zeit [h]** | **NH₃ [Eq.] ^{e)}** | **Reatkionsdruck [bar]** | **Metallsalz [M]** | **Met. [M] (mol%)^{f)}** | **Ligand [L]** | **Lig. [L] (mol%)^{f)}** | **Umsatz ^{b}** | **Selekti ivität^{c}** | | |
|---|---|---|---|---|---|---|---|---|---|---|---|---|---|
| | | | | | | | | | | | **a** | **: b** | **: c** |
| 1 | Toluol | 180 | 12 | 6 | 65^{d)} | [RuHCl(CO)(PPh₃)₃] | 0,2 | Triphos | 0,2 | 97,6 | 26,4 | 13,4 | 54,0 |
| 2 | Toluol | 155 | 12 | 6 | 40 | [RuHCl(CO)(PPh₃)₃] | 0,2 | DPPEPP | 0,2 | 21,5 | 46,0 | 0,0 | 2,3 |
| a) 50ml Lösungsmittel; Ansatzgröße: 25 mmol Diethylenglykol b) Auswertung per GC (FI.%); c) Produktselektivität bestimmt per GC; d) kalt aufgepresst: 5 bar H₂, 8 bar NH₃, e) Molare Equivalente NH₃ pro OH-Funktion am Substrat; f) mol% bezogen auf Anzahl der OH-Funktionen am Substrat; | | | | | | | | | | | | | |

**Tabelle 4: Aminierung von Diethylenglykol mit XIVb als Katalysatorsystem**

| | | | | | | | | | | |
|---|---|---|---|---|---|---|---|---|---|---|
| 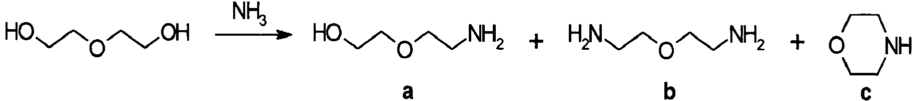 | | | | | | | | | | |

| **Nr^{a)}** | **Lösungsmittel** | **T [°C]** | **Zeit [h]** | **NH₃ [Eq.]^{d)}** | **Reaktionsdruck [bar]** | **Weitere Bedingungen** | **Umsatz^{b)}** | **Selektivität^{c)}** | | |
|---|---|---|---|---|---|---|---|---|---|---|
| | | | | | | | | **a** | **b** | **c** |
| 1 | Toluol | 135 | 12 | 6 | 38 | | 40,4 | 85,8 | 2,5 | 6,7 |
| 2 | Toluol | 135 | 12 | 6 | 38 | 0,2 mol% KOtBu | 11,7 | 69,8 | 3,4 | 5,0 |
| 3 | Toluol | 135 | 12 | 6 | 36 | 1 mol% Wasser | 37,9 | 86,4 | 2,1 | 6,0 |
| 4 | Toluol | 135 | 12 | 6 | 37 | | 42,1 | 87,1 | 3,3 | 6,5 |
| 5 | Toluol | 135 | 12 | 9 | 42 | | 33,8 | 81,4 | 1,5 | 5,5 |
| 6 | Toluol | 135 | 12 | 18 | 57 | | 36,5 | 78,4 | 2,9 | 9,4 |
| 7 | Toluol | 135 | 15 | 1.1 | 9 | | 49,1 | 76,4 | 3,2 | 8,7 |
| 8 | Toluol | 135 | 24 | 6 | 37 | | 60,9 | 75,8 | 9,3 | 8,3 |
| 9 | Toluol | 135 | 60 | 9 | 45 | Kat: 0.05 mol% | 28,1 | 81,7 | 6,3 | 2,5 |
| 10 | Toluol | 155 | 12 | 6 | 40 | 5,0 mol% Wasser | 74,8 | 57,2 | 18,5 | 11,1 |
| 11 | Toluol | 155 | 12 | 6 | 66 | 5 bar H2 | 61,8 | 69,2 | 18,6 | 8,0 |
| 12 | Toluol | 155 | 12 | 9 | 63 | 5 bar H2 + 1,0 mol% Wasser | 55,5 | 72,7 | 16,0 | 6,8 |
| 13 | Toluol | 155 | 12 | 9 | 66 | 5 bar H2 | 53,1 | 75,0 | 14,1 | 5,8 |
| 14 | p-Xylol | 155 | 12 | 6 | 48 | | 74,4 | 65,8 | 11,5 | 9,5 |
| 15 | p-Xylol | 155 | 12 | 6 | 38 | 1,0 mol% Wasser | 77,5 | 52,9 | 21,6 | 16,9 |
| 16 | p-Xylol | 155 | 24 | 6 | 53 | 1,0 mol% Wasser | 84,6 | 51,8 | 20,8 | 12,9 |
| 17 | p-Xylol | 180 | 12 | 6 | 50 | | 100,0 | 0,4 | 46,1 | 27,9 |
| 18 | p-Xylol | 180 | 12 | 6 | 50 | 5mol% H2O | 100,0 | 0,4 | 48,2 | 27,4 |
| a) Bedingungen wenn nicht anders angegeben: 50 ml Lösungsmittel, Ansatzgröße 25 mmol Diethylenglykol, 0.1 mol% Katalysatorkomplex XIVb (bzgl. Anzahl der OH-Funktionen am Substrat), b) Auswertung per GC (FI.%), c) Produktselektivität bestimmt per GC,d) Molare Equivalente NH₃ pro OH-Funktion am Substrat; | | | | | | | | | | |

**Tabelle 5: Aminierung von Monoethylenglykol mit XIVb als Katalysatorsystem**

| | | | | | | | | | | |
|---|---|---|---|---|---|---|---|---|---|---|
| 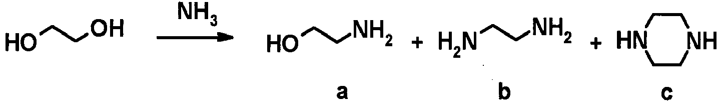 | | | | | | | | | | |

| **Nr^{a)}** | **Lösungsmittel** | **T[°C]** | **Zeit [h]** | **NH₃ [Eq.]^{d)}** | **Reaktionsdruck [bar]** | **Weitere Bedingungen** | **Umsatz^{b}** | **Selektivität** | | |
|---|---|---|---|---|---|---|---|---|---|---|
| | | | | | | | | **a** | **b** | **c** |
| 1 | Toluol | 135 | 12 | 6 | 35 | | 14,0 | 68,6 | 16,6 | 0,5 |
| 2 | Toluol | 135 | 12 | 6 | 38 | 0.2 mol% KOtBu | 39,3 | 65,8 | 18,9 | 0,7 |
| 3 | Toluol | 135 | 12 | 9 | 44 | | 11,0 | 71,9 | 17,5 | 0,7 |
| 4 | Toluol | 135 | 12 | 12 | 48 | | 10,6 | 68,2 | 17,5 | 2,5 |
| 5 | Toluol | 135 | 12 | 18 | 54 | | 13,7 | 69,0 | 15,6 | 2,7 |
| 6 | p-Xylol | 155 | 3 | 6 | 38 | | 18,2 | 56,9 | 19,5 | 1,0 |
| a) Bedingungen wenn nicht anders angegeben: 50 ml Lösungsmittel, Ansatzgröße 25 mmol Monoethylenglykol, 0.1 mol% Katalysatorkomplex XIVb (bzgl. Anzahl der OH-Funktionen am Substrat), b) Auswertung per GC (FI.%), c) Produktselektivität bestimmt per GC, d) Molare Equivalente NH₃ pro OH-Funktion am Substrat; | | | | | | | | | | |

**Tabelle 6: Aminierung von Diethanolamin mit XIVb ais Katalysatorsystem**

| | | | | | | | | | | | |
|---|---|---|---|---|---|---|---|---|---|---|---|
| 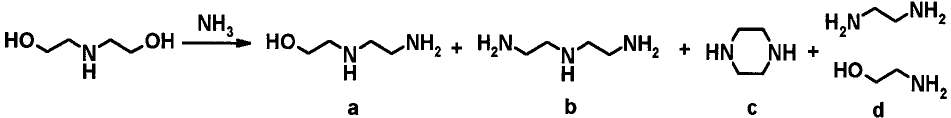 | | | | | | | | | | | |

| **Nr^{a)}** | **Lösungsmittel** | **T [°C]** | **Zeit [h]** | **NH₃ [Eq.]^{d)}** | **Reaktionsdruck [bar]** | **Weitere Bedingungen** | **Umsatz^{b}** | **Selektivität** | | | |
|---|---|---|---|---|---|---|---|---|---|---|---|
| | | | | | | | | **a** | **b** | **c** | **d** |
| 1 | Toluol | 135 | 12 | 9 | 43 | | 22,7 | 51,9 | 0,4 | 0,0 | 31,2 |
| 2 | Toluol | 155 | 12 | 6 | 44 | | 49,0 | 41,4 | 1,8 | 0,0 | 30,2 |
| 3 | Toluol | 155 | 24 | 6 | 42 | | 69,0 | 32,6 | 2,1 | 0,0 | 30,6 |
| 4 | Toluol | 155 | 12 | 6 | 45 | | 32,5 | 31,8 | 1,8 | 0,0 | 34,6 |
| 5 | Toluol | 155 | 12 | 9 | 54 | | 57,5 | 47,4 | 2,5 | 4,1 | 34,3 |
| 6 | Toluol | 155 | 12 | 6 | 44 | 1 mol% KOtBu | 25,7 | 34,8 | 3,3 | 0,0 | 25,3 |
| 7 | Toluol | 155 | 12 | 12 | 57 | | 50,8 | 39,8 | 1,3 | 3,0 | 36,6 |
| 8 | Toluol | 155 | 12 | 6 | 44 | 1 mol% Wasser | 51,7 | 40,5 | 1,4 | 3,4 | 33,8 |
| 9 | Toluol | 155 | 12 | 6 | 43 | 5mol% Wasser | 50,6 | 42,2 | 1,4 | 4,6 | 30,8 |
| 10 | Toluol | 155 | 12 | 6 | 60 | 5bar H2 | 33,4 | 51,1 | 1,4 | 4,4 | 30,6 |
| a) Bedingungen wenn nicht anders angegeben: 50 ml Lösungsmittel, Ansatzgröße 25 mmol Diethanolamin, 0.1 mol% Katalysatorkomplex XIVb (bzgl. Anzahl der OH-Gruppen am Substrat), b) Auswertung per GC (FI.%), c) Produktselektivität bestimmt per GC, d) Molare Equivalente NH₃ pro OH-Funktion am Substrat; | | | | | | | | | | | |

**Tabelle 7: Aminierung von (1,5-Pentandiol, 1,6-Hexandiol, 1,10-Decandiol 1,9-Nonandiol) mit verschiedenen Katalysatorsystemen**

| | | | | | | | | | | | | | |
|---|---|---|---|---|---|---|---|---|---|---|---|---|---|
| 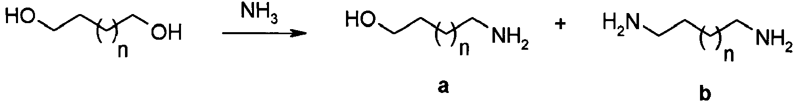 | | | | | | | | | | | | | |

| **Nr^{a)}** | **Substrat** | **T [°C]** | **Reaktionszeit [h]** | **NH₃ [Eq.]^{e)}** | **Reaktionsdruck [bar]** | **Lösungsmittel (wasserfrei)** | **Metallsalz [M]** | **Met. [M] (mol%)^{f)}** | **Ligand [L]** | **Lig. [L] (mol%) ^{f)}** | **Umsatz ^{b)}** | **Selektivität^{c}** | |
|---|---|---|---|---|---|---|---|---|---|---|---|---|---|
| | | | | | | | | | | | | **a** | **: b** |
| 1 | 1,6-Hexandiol | 155 | 12 | 6 | 42 | Toluol | [RuHCl(CO)(PPh₃)₃] | 0,10 | Triphos | 0,10 | 83,0 | 61,3 | 25,7 |
| 2 | 1,6-Hexandiol | 155 | 12 | 6 | 36 | Toluol | [RuHCl(CO)(PPh₃)₃] | 0,10 | Xantphos | 0,10 | 33,4 | 84,9 | 4,6 |
| 3 | 1,6-Hexandiol | 155 | 12 | 6 | 40 | Toluol | [RuHCl(CO)(PPh₃)_{3]} | 0,10 | DPPEPP | 0,10 | 70,7 | 66,5 | 16,0 |
| 4 | 1,6-Hexandiol | 155 | 12 | 6 | 44 | Toluol | [RuHCl(CO)(PPh₃)₃] | 0,10 | Rhodaphos | 0,10 | 35,1 | 53,0 | 2,0 |
| 5 | 1,10-Decandiol | 155 | 24 | 6 | 39 | Toluol | [RuHCl(CO)(PPh₃)₃] | 0,20 | Triphos | 0,20 | 85,7 | 43,0 | 44,4 |
| 7 | 1,5-Pentandiol | 155 | 12 | 6 | 40 | Toluol | [RuHCl(CO)(PPh₃)₃] | 0,10 | Triphos | 0,10 | 70,3 | 66,8 | 1,3 |
| 8 | 1,5-Pentandiol | 155 | 12 | 6 | 45 | Toluol | [RuHCl(CO)(PPh₃)₃] | 0,10 | DPPEPP | 0,10 | 50,9 | 64,6 | 7,1 |
| 9^{d)} | 1,9-Nonandiol | 155 | 24 | 12 | 14 | Mesitylen | [RuHCl(CO)(PPh₃)₃] | 0,20 | Triphos | 0,20 | 79,3 | 54,0 | 31,1 |
| a) 50ml Lösungsmittel; Ansatzgröße: 25 mmol Diol; b) Auswertung per GC (FI.%); c) Produktselektivität bestimmt per GC (FI.%); d) Ansatzgröße: 50 mmol Substrat; Ansatzgröße: 50 mmol Diol; e) Molare Equivalente NH₃ pro OH-Funktion am Substrat; f) mol% bezogen auf Anzahl der OH-Funktionen am Substrat; | | | | | | | | | | | | | |

**Tabelle 8: Aminierung von 2,5- Dimethanolfuran**

| | | | | | | | | | | | | | | |
|---|---|---|---|---|---|---|---|---|---|---|---|---|---|---|
| 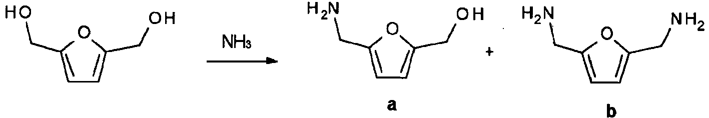 | | | | | | | | | | | | | | |

| **Nr^{a)}** | **Substrat** | **T [°C]** | **Reaktionszeit [h]** | **konz. [mol/l]** | **NH₃ [Eq.]^{d)}** | **Reaktionsdruck [bar]** | **Lösungsmittel (wasserfrei)** | **Metallsalz [M]** | **Met. [M] (mol%)^{e)}** | **Ligand [L]** | **Lig. [L] (mol%)^{e)}** | **Umsatz ^{b)}** | **Selektivität ^{c}** | |
|---|---|---|---|---|---|---|---|---|---|---|---|---|---|---|
| | | | | | | | | | | | | | **a** | **: b** |
| 1 | 2,5-Dimethanolfuran | 140 | 24 | 1 | 6 | 15 | THF | [RuHCl(CO)(PPh₃)₃] | 0,20 | Triphos | 0,20 | 46,8 | 63,1 | 10,2 |
| 2 | 2,5-Dimethanolfuran | 140 | 3 | 0,5 | 6 | 32 | tert-Butanol | XIVb | 0,10 | - | - | 84,6 | 49,2 | 43,6 |
| a) 40ml Lösungsmittel; Ansatzgröße: 40 mmol Diol; b) Auswertung per GC (FI.%); c) Produktselektivität bestimmt per GC (FI.%); d) Molare Equivalente NH₃ pro OH-Funktion am Substrat; e) mol% bezogen auf Anzahl der OH-Funktionen am Substrat; | | | | | | | | | | | | | | |

## Patentansprüche

1. Verfahren zur Herstellung von Alkanolaminen, die eine primäre Aminogruppe (-NH₂) und eine Hydroxylgruppe (-OH) aufweisen, durch Alkoholaminierung von Diolen, die zwei Hydroxylgruppe (-OH) aufweisen, mit Ammoniak unter Wasserabspaltung, wobei die Reaktion homogen-katalysiert in Gegenwart mindestens eines Komplexkatalysators, der mindestens ein Element ausgewählt aus den Gruppen 8, 9 und 10 des Periodensystems sowie mindestens einen Phosphordonorliganden enthält, durchgeführt wird.

2. Verfahren nach Anspruch 1, wobei der Komplexkatalysator ein Katalysator der Formel (I) ist: 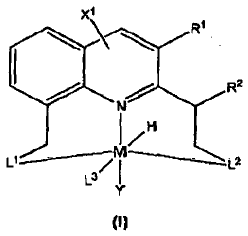 in der
L¹ und L² unabhängig voneinander PR^{a}R^{b}, NR^{a}R^{b}, Sulfid, SH, S(=O)R, C₅-C₁₀-Heteroaryl enthaltend mindestens ein Heteroatom ausgewählt aus N, O und S, AsR^{a}R^{b}, SbR^{a}R^{b} oder N-heterocyclische Carbene der Formel (II) oder (III): 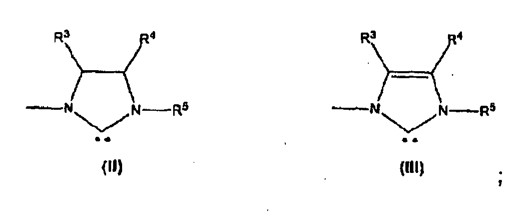
R3 R4 R3 R4

N N—R5 N N—R5
(II) (III)
;
L³ einzähniger Zweielektronendonor ausgewählt aus der Gruppe CO, PR^{a}R^{b}R^{c}, NO⁺, AsR^{a}R^{b}R^{c}, SbR^{a}R^{b}R^{c}, SR^{a}R^{b}, RCN, RNC, N₂, PF₃, CS, Pyridin, Thiophen, Tetrahydrothiophen und N-heterocyclische Carbene der Formel (II) oder (III);
R¹ und R² beide Wasserstoff oder gemeinsam mit den Kohlenstoffatomen, an die sie gebunden sind, ein Phenylring, der zusammen mit der Chinolinyleinheit der Formel (I) eine Acridinyleinheit bildet;
R, R^{a}, R^{b}, R^{c}, R³, R⁴, und R⁵ unabhängig voneinander unsubstituiertes oder zumindest monosubstituiertes C₁-C₁₀-Alkyl, C₃-C₁₀-Cycloalkyl, C₃-C₁₀-Heterocyclyl enthaltend mindestens ein Heteroatom ausgewählt aus N, O und S, C₅-C₁₀-Aryl oder C₅-C₁₀-Heteroaryl enthaltend mindestens ein Heteroatom ausgewählt aus N, O und S,
wobei die Substituenten ausgewählt sind aus der Gruppe bestehend aus: F, Cl, Br, OH. CN, NH₂ und C₁-C₁₀-Alkyl;
Y monoanionischer Ligand ausgewählt aus der Gruppe H, F, Cl, Br, I, OCOR, OCOCF₃, OSO₂R, OSO₂CF₃, CN, OH, OR und N(R)₂ oder neutrale Moleküle ausgewählt aus der Gruppe NH₃, N(R)₃ und R₂NSO₂R;
X¹ ein, zwei, drei, vier, fünf, sechs, oder sieben Substituenten an einem oder mehreren Atomen der Acridinyleinheit oder ein, zwei, drei, vier oder fünf Substituenten an einem oder mehreren Atomen der Chinolinyleinheit,
wobei X¹ unabhängig voneinander ausgewählt ist aus der Gruppe bestehend aus Wasserstoff, F, Cl, Br, I, OH, NH₂, NO₂, -NC(O)R, C(O)NR₂, -OC(O)R, -C(O)OR, CN und Boranderivaten, die aus dem Katalysator der Formel (I) durch Reaktion mit NaBH₄ erhältlich sind und unsubstituiertem oder zumindest monosubstituiertem C₁-C₁₀-Alkoxy, C₁-C₁₀-Alkyl, C₃-C₁₀-Cycloalkyl, C₃-C₁₀-Heterocyclyl enthaltend mindestens ein Heteroatom ausgewählt aus N, O und S, C₅-C₁₀-Aryl und C₅-C₁₀-Heteroaryl enthaltend mindestens ein Heteroatom ausgewählt aus N, O und S,
wobei die Substituenten ausgewählt sind aus der Gruppe bestehend aus: F, Cl, Br, OH, CN, NH₂ und C₁-C₁₀-Alkyl;
und
M Eisen, Cobalt, Nickel, Ruthenium, Rhodium, Palladium, Osmium, Iridium oder Platin
bedeuten.

3. Verfahren nach Anspruch 1 oder 2, wobei R¹ und R² beide Wasserstoff sind und der Komplexkatolysator ein Katalysator der Formel (IV) ist: 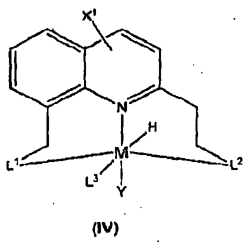 und X¹, L¹, L², L³ und Y die in Anspruch 2 genannten Bedeutungen aufweisen.

4. Verfahren nach Anspruch 1 oder 2, wobei R¹ und R² gemeinsam mit den Kohlenstoffatomen, an die sie gebunden sind, ein Phenylring bilden, der zusammen mit der Chinolinyleinheit der Formel (I) eine Acridinyleinheit bildet und der Komplexkatalysator ein Katalysator der Formel (V) ist: 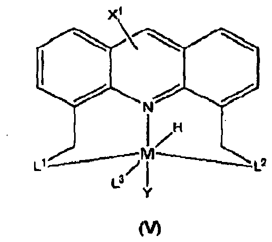 und X', L¹, L², L³ und Y die in Anspruch 2 genannten Bedeutungen aufweisen.

5. Verfahren nach Anspruch 1 oder 2, wobei der Komplexkatalysator aus der Gruppe der Katalysatoren der Formel (VI), (VII), (VIII), (IX), (X), (XI), (XII) und (XIII) ausgewählt ist: 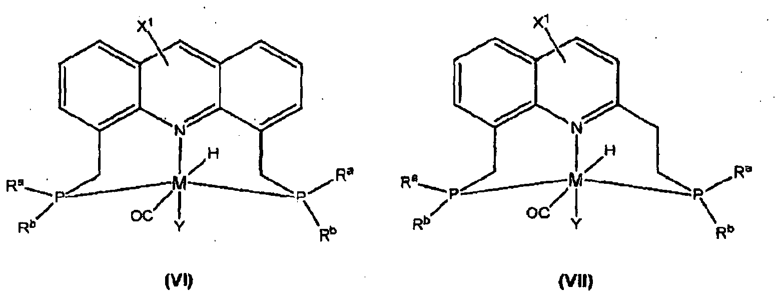 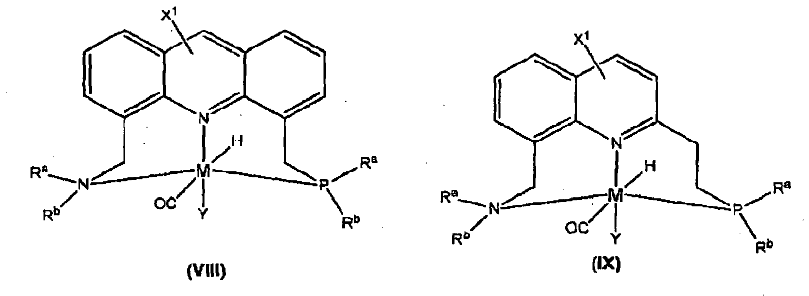 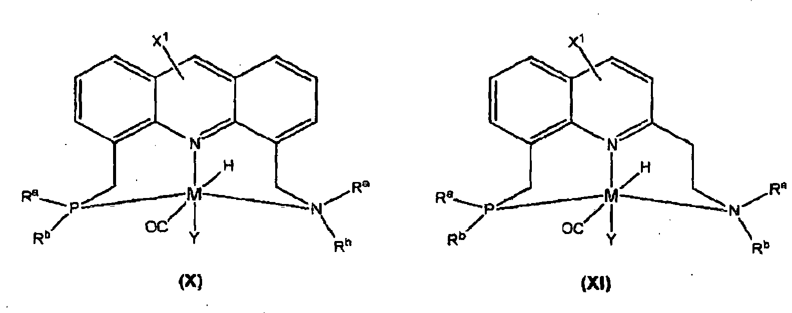 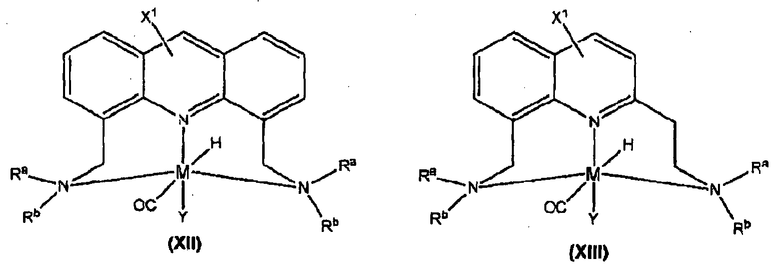 und X¹, R^{a}, R^{b}, und Y die in Anspruch 2 genannten Bedeutungen aufweisen.

6. Verfahren nach Anspruch 1 oder 2, wobei der Komplexkatalysator ein Katalysator der Formel (XIVa) ist:

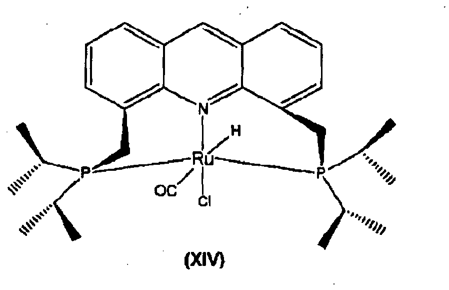

7. Verfahren nach Anspruch 1 oder 2, wobei der Komplexkatalysator ein Katalysator der Formel (XIVb) ist:

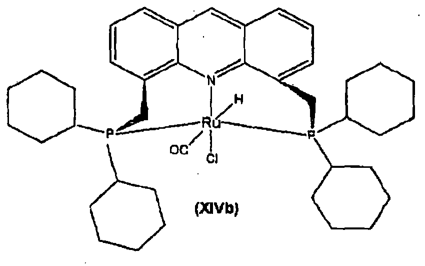

8. Verfahren nach Anspruch 1, wobei der Komplexkatalysator ein Katalysator der Formel (XV) ist: 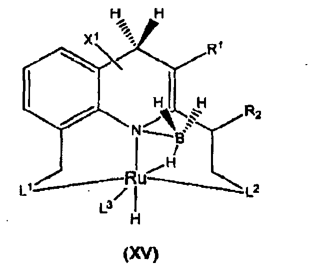 in der
L¹ und L² unabhängig voneinander PR^{a}R^{b}, NR^{a}R^{b}, Sulfid, SH, S(=O)R, C₅-C₁₀-Hetercaryl enthaltend mindestens ein Heteroatom ausgewählt aus N, O und S, AsR^{a}R^{b}, SbR^{a}R^{b} oder N-heterocyclische Carbene der Formel (II) oder (III): 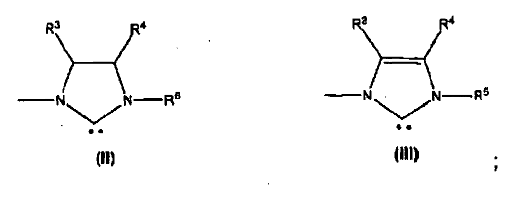
L³ einzähniger Zweielektronendonor ausgewählt aus der Gruppe CO, PR^{a}R^{b}R^{c}, NO⁺, AsR^{a}R^{b}R^{c}, SbR^{a}R^{b}R^{c}, SR^{a}R^{b}, RCN, RNC, N₂, PF₃, CS, Pyridin, Thiophen, Tetrahydrothiophen und N-heterocyclische Carbene der Formel (II) oder (III);
R¹ und R² beide Wasserstoff oder gemeinsam mit den Kohlenstoffatomen, an die sie gebunden sind, ein Phenylring, der zusammen mit der Chinolinyleinheit der Formel (I) eine Acridinyleinheit bildet;
R, R^{a}, R^{b}, R^{c}, R³, R⁴, und R⁵ unabhängig voneinander unsubstituiertes oder zumindest monosubstituiertes C₁-C₁₀-Alkyl, C₃-C₁₀-Cycloalkyl, C₃-C₁₀-Heterocyclyl enthaltend mindestens ein Heteroatom ausgewählt aus N, O und S, C₅-C₁₀-Aryl oder C₅-C₁₀-Heteroaryl enthaltend mindestens ein Heteroatom ausgewählt aus N, O und S,
wobei die Substituenten ausgewählt sind aus der Gruppe bestehend aus: F, Cl, Br, OH, CN, NH₂ und C₁-C₁₀-Alkyl;
Y monoanionischer Ligand ausgewählt aus der Gruppe H, F, Cl, Br, I, OCOR, OCOCF₃, OSO₂R, OSO₂CF₃, CN, OH, OR und N(R)₂ oder neutrale Moleküle ausgewählt aus der Gruppe NH₃, N(R)₃ und R₂NSO₂R;
X¹ ein, zwei, drei, vier, fünf, sechs, oder sieben Substituenten an einem oder mehreren Atomen der Acridinyleinheit oder ein, zwei, drei, vier oder fünf Substituenten an einem oder mehreren Atomen der Chinolinyleinheit,
wobei X¹ unabhängig voneinander ausgewählt ist aus der Gruppe bestehend aus Wasserstoff, F, Cl, Br, I, OH, NH₂, NO₂, -NC(O)R, C(O)NR₂, -OC(O)R, -C(O)OR, CN und Boranderivaten, die aus dem Katalysator der Formel (I) durch Reaktion mit NaBH₄ erhältlich sind und unsubstituiertem oder zumindest monosubstituiertern C₁-C₁₀-Alkoxy, C₁-C₁₀-Alkyl, C₃-C₁₀-Cycloslkyl, C₃-C₁₀-Heterocyclyl enthaltend mindestens ein Heteroatom ausgewählt aus N, O und S, C₅-C₁₀-Aryl und C₅-C₁₀-Meteroaryl enthaltend mindestens ein Heteroatom ausgewählt aus N, O und S,
wobei die Substituenten ausgewählt sind aus der Gruppe bestehend aus: F, Cl, Br, OH, CN, NH₂ und C₁-C₁₀-Alkyl;
und
M Eisen, Cobalt, Nickel, Ruthenium, Rhodium, Palladium, Osmium, Iridium oder Platin
bedeuten.

9. Verfahren nach einem der Ansprüche 1 oder 8, wobei der Komplexkatalysator ein Katalysator der Formel (XVI) ist:

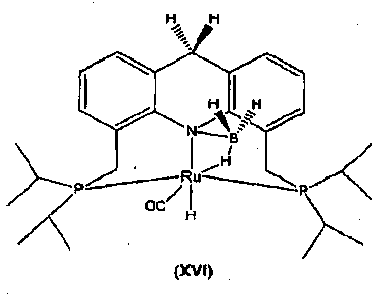

10. Verfahren nach einem der Ansprüche 1 bis 5, wobei im Komplexkatalysator Y ausgewählt ist aus F, Cl und Br.

11. Verfahren nach einem der Ansprüche 2 bis 10, wobei im Komplexkatalysator L³ CO ist.

12. Verfahren nach Anspruch 1, wobei die Alkoholaminierung in Gegenwart mindestens eines Komplexkatalysators, der mindestens ein Element ausgewählt aus den Gruppen 8, 9 und 10 des Periodensystems sowie mindestens einen Phosphordonorliganden der allgemeinen Formel (XXI) enthält, 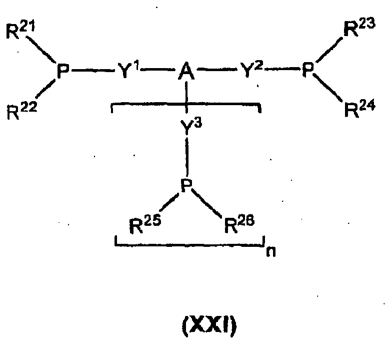 durchgeführt wird, wobei
n ist 0 oder 1,
R²¹, R²², R²³, R²⁴, R²⁵, R²⁶ sind unabhängig voneinander unsubstituiertes oder zumindest monosubstituiertes C₁-C₁₀-Alkyl, C₁-C₄-Alkyldiphenylphosphin (-C₁-C₄-Alkyl-P(Phenyl)₂), C₃-C₁₀-Cycloalkyl, C₃-C₁₀-Heterocyclyl enthaltend mindestens ein Heteroatom ausgewählt aus N, O und S, C₅-C₁₄-Aryl oder C₅-C₁₀-Heteroaryl enthaltend mindestens ein Heteroatom ausgewählt aus N, Ö und S,
wobei die Substituenten ausgewählt sind aus der Gruppe bestehend aus: F, Cl, Br, OH, CN, NH₂ und C₁-C₁₀-Alkyl;
A ist
i) eine Brückengruppe ausgewählt aus der Gruppe unsubstituiertes oder zumindest monosubstituiertes N, O, P, C₁-C₆-Alkan, C₃-C₁₀-Cycloalkan, C₃-C₁₀-Heterocycloalkan enthaltend mindestens ein Heteroatom ausgewählt aus N, O und S, C₅-C₁₄-Aromat oder C₅-C₆-Heteroaromat enthaltend mindestens ein Heteroatom ausgewählt aus N, O und S,
wobei die Substituenten ausgewählt sind aus der Gruppe bestehend aus: C₁-C₄-Alkyl, Phenyl, F, Cl, Br, OH, OR²⁷, NH₂, NHR²⁷ oder N(R²⁷)₂,
wobei R²⁷ausgewählt ist aus C₁-C₁₀-Alkyl und C₅-C₁₀-Aryl;
oder
ii) eine Brückengruppe der Formel (XXII) oder (XXIII): 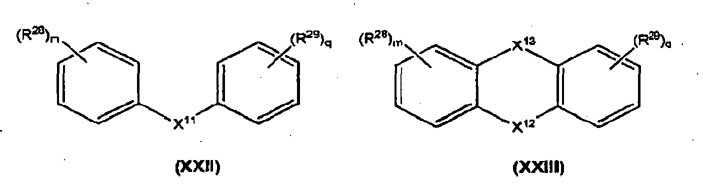
m, q sind unabhängig voneinander 0, 1, 2, 3 oder 4,
R²⁸, R²⁹ sind unabhängig voneinander ausgewählt aus der Gruppe C₁-C₁₀-Alkyl, F, Cl, Br, OH, OR²⁷, NH₂, NHR²⁷ und N(R²⁷)₂,
wobei R²⁷ ausgewählt ist aus C₁-C₁₀-Alkyl und C₅-C₁₀-Aryl;
X¹¹, X¹² sind unabhängig voneinander NH, O oder S,
X¹³ ist eine Bindung, NH, NR³⁰, O, S oder CR³¹R³²,
R³⁰ ist unsubstituiertes oder zumindest monosubstituiertes C₁-C₁₀-Akyl, C₃-C₁₀-Cycloalkyl, C₃-C₁₀-Heterocyclyl enthaltend mindestens ein Heteroatom ausgewählt aus N, O und S, C₅-C₁₄-Aryl oder C₅-C₁₀-Heteroaryl enthaltend mindestens ein Heteroatom ausgewählt aus N, O und S,
wobei die Substituenten ausgewählt sind aus der Gruppe bestehend aus: F, Cl, Br, OH, CN, NH₂ und C₁-C₁₀-Alkyl;
R³¹, R³² sind unabhängig voneinander unsubstituiertes oder zumindest monosubstituiertes C₁-C₁₀-Alkyl, C₁-C₁₀-Alkoxy, C₃-C₁₀-Cycloalkyl, C₃-C₁₀-Cycloalkoxy,C₃-C₁₀-Heterocyclyl enthaltend mindestens ein Heteroatom ausgewählt aus N, O und S, C₅-C₁₄-Aryl, C₅-C₁₄-Aryloxy oder C₅-C₁₀-Heteroaryl enthaltend mindestens ein Heteroatom ausgewählt aus N, O und S,
wobei die Substituenten ausgewählt sind aus der Gruppe bestehend aus: F, Cl, Br, OH, CN, NH₂ und C₁-C₁₀-Alkyl;
Y¹, Y², Y³ sind unabhängig voneinander eine Bindung, unsubstituiertes oder zumindest monosubstituiertes Methylen, Ethylen, Trimethylen, Tetramethylen, Pentamethylen oder Hexamethylen,
wobei die Substituenten ausgewählt sind aus der Gruppe bestehend aus: F, Cl, Br, OH, OR²⁷, CN, NH₂, NHR²⁷, N(R²⁷)₂ und C₁-C₁₀₋Alkyl,
wobei R²⁷ ausgewählt ist aus C₁-C₁₀-Alkyl und C₅-C₁₀-Aryl.

13. Verfahren nach einem der Ansprüche 1 bis 11, wobei das Diol zwei funktionelle Gruppen der Formeln (-CH₂-OH) aufweist.

14. Verfahren nach einem der Ansprüche 1 bis 13, wobei die Herstellung der Alkanolamlne bei einer Temperatur von 110 bis 160 °C und einem Druck von 1 bis 15 MPa durchgeführt wird.

15. Verwendung eines Komplexkatalysators, der mindestens ein Element ausgewählt aus den Gruppen 8, 9 und 10 des Periodensystems sowie mindestens einen Phosphordonorllganden enthält, zur homogen-katalysierten Herstellung von Alkanolaminen, die eine primäre Aminogruppe (-NH₂) und eine Hydroxylgruppe (-OH) aufweisen, durch Alkoholaminierung von Diolen, die zwei Hydroxylgruppen (-OH) aufweisen, mit Ammoniak.

## Claims

1. A process for preparing alkanolamines which have a primary amino group (-NH₂) and a hydroxyl group (-OH) by alcohol amination of diols having two hydroxyl groups (-OH) by means of ammonia with elimination of water, wherein the reaction is carried out homogeneously catalyzed in the presence of at least one complex catalyst comprising at least one element selected from groups 8, 9 and 10 of the Periodic Table and also at least one phosphorus donor ligand.

2. The process according to claim 1, wherein the complex catalyst is a catalyst of the formula (I): 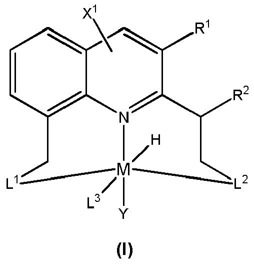 where
L¹ and L² are each, independently of one another, PR^{a}R^{b}, NR^{a}R^{b}, sulfide, SH, S(=O)R, C₅-C₁₀-heteroaryl comprising at least one heteroatom selected from among N, 0 and S, AsR^{a}R^{b}, SbR^{a}R^{b} and N-heterocyclic carbenes of the formula (II) or (III): 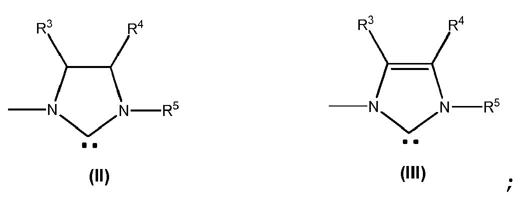
L³ is a monodentate two-electron donor selected from the group consisting of CO, PR^{a}R^{b}R^{c}, NO⁺, AsR^{a}R^{b}R^{c}, SbR^{a}R^{b}R^{c}, SR^{a}R^{b}, RCN, RNC, N₂, PF₃, CS, pyridine, thiophene, tetrahydrothiophene and N-heterocyclic carbenes of the formula (II) or (III);
R¹ and R² are both hydrogen or together with the carbon atoms to which they are bound form a phenyl ring which together with the quinolinyl unit of the formula (I) forms an acridinyl unit;
R, R^{a}, R^{b}, R^{c}, R³, R⁴ and R⁵ are each, independently of one another, unsubstituted or at least monosubstituted C₁-C₁-alkyl, C₃-C₁₀-cycloalkyl, C₃-C₁₀-heterocyclyl comprising at least one heteroatom selected from among N, 0 and S, C₅-C₁₀-aryl or C₅-C₁₀-heteroaryl comprising at least one heteroatom selected from among N, 0 and S,
where the substituents are selected from the group consisting of: F, Cl, Br, OH, CN, NH₂ and C₁-C₁₀-alkyl;
Y is a monoanionic ligand selected from the group consisting of H, F, Cl, Br, I, OCOR, OCOCF₃, OSO₂R, OSO₂CF₃, CN, OH, OR and N(R)₂ or an uncharged molecule selected from the group consisting of NH₃, N(R)₃ and R₂NSO₂R;
X¹ represents one, two, three, four, five, six or seven substituents on one or more atoms of the acridinyl unit or one, two, three, four or five substituents on one or more atoms of the quinolinyl unit,
where the radicals X¹ are selected independently from the group consisting of hydrogen, F, Cl, Br, I, OH, NH₂, NO₂, - NC(O)R, C(O)NR₂, -OC(O)R, -C(O)OR, CN and borane derivatives which can be obtained from the catalyst of the formula (I) by reaction with NaBH₄ and unsubstituted or at least monosubstituted C₁-C₁₀-alkoxy, C₁-C₁₀-alkyl, C₃-C₁₀-cycloalkyl, C₃-C₁₀-heterocyclyl comprising at least one heteroatom selected from among N, 0 and S, C₅-C₁₀-aryl and C₅-C₁₀-heteroaryl comprising at least one heteroatom selected from among N, 0 and S,
where the substitutents are selected from the group consisting of: F, Cl, Br, OH, CN, NH₂ and C₁-C₁₀-alkyl;
and
M is iron, cobalt, nickel, ruthenium, rhodium, palladium, osmium, iridium or platinum.

3. The process according to claim 1 or 2, wherein R¹ and R² are both hydrogen and the complex catalyst is a catalyst of the formula (IV): 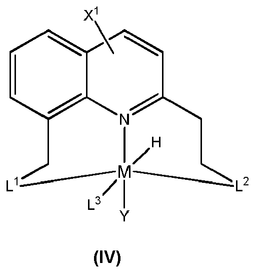 and X¹, L¹, L², L³ and Y are as defined in claim 2.

4. The process according to claim 1 or 2, wherein R¹ and R² together with the carbon atoms to which they are bound form a phenyl ring which together with the quinolinyl units of the formula (I) forms an acridinyl unit and the complex catalyst is a catalyst of the formula (V): 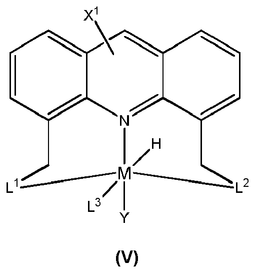 and X¹, L¹, L², L³ and Y are as defined in claim 2.

5. The process according to claim 1 or 2, wherein the complex catalyst is selected from the group of catalysts of the formulae (VI), (VII), (VIII), (IX), (X), (XI), (XII) and (XIII): 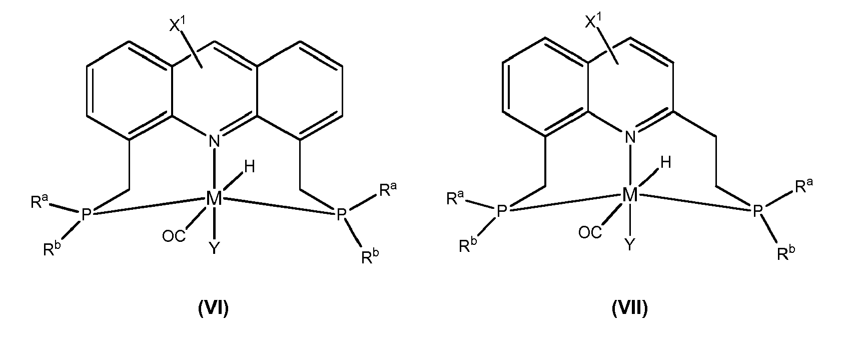 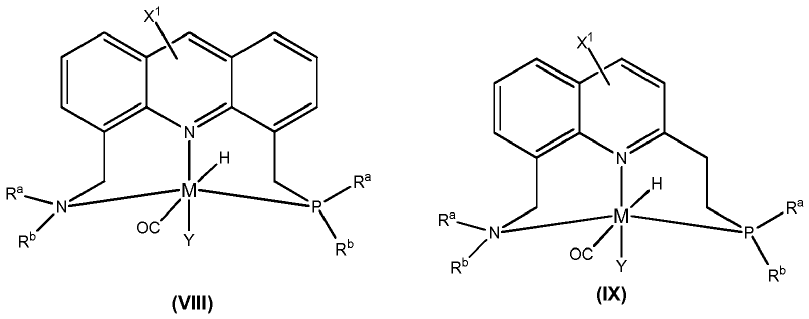 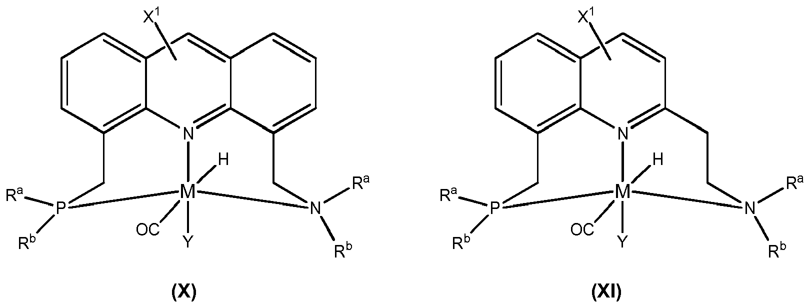 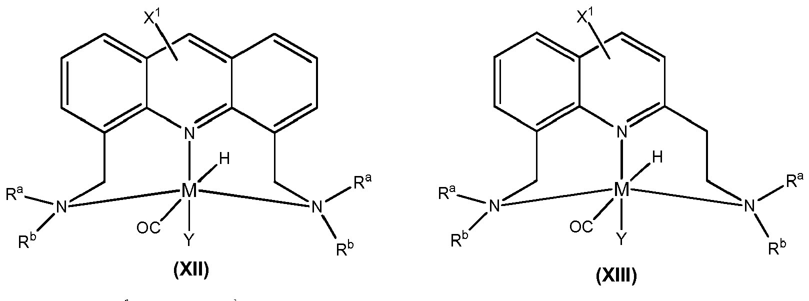 and X¹, R^{a}, R^{b} and Y are as defined in claim 2.

6. The process according to claim 1 or 2, wherein the complex catalyst is a catalyst of the formula (XIVa) :

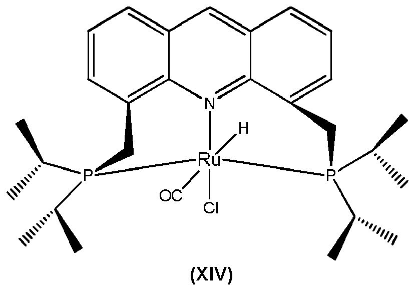

7. The process according to claim 1 or 2, wherein the complex catalyst is a catalyst of the formula (XIVb) :

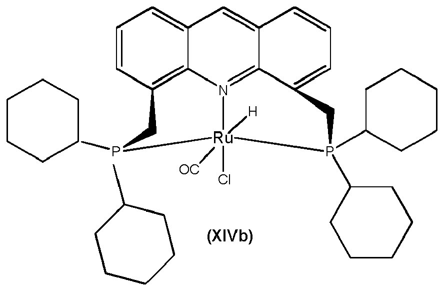

8. The process according to claim 1, wherein the complex catalyst is a catalyst of the formula (XV): 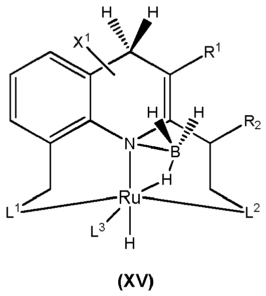 where
L¹ and L² are each, independently of one another, PR^{a}R^{b}, NR^{a}R^{b}, sulfide, SH, S(=O)R, C₅-C₁₀-heteroaryl comprising at least one heteroatom selected from among N, 0 and S, AsR^{a}R^{b}, SbR^{a}R^{b} or N-heterocyclic carbenes of the formula (II) or (III): 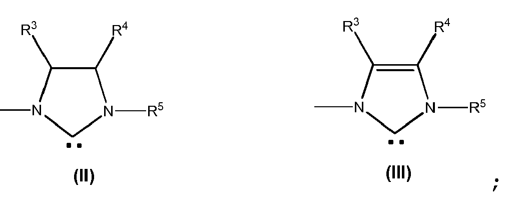
L³ is a monodentate two-electron donor selected from the group consisting of CO, PR^{a}R^{b}R^{c}, NO⁺, AsR^{a}R^{b}R^{c}, SbR^{a}R^{b}R^{c}, SR^{a}R^{b}, RCN, RNC, N₂, PF₃, CS, pyridine, thiophene, tetrahydrothiophene and N-heterocyclic carbenes of the formula (II) or (III);
R¹ and R² are both hydrogen or together with the carbon atoms to which they are bound form a phenyl ring which together with the quinolinyl unit of the formula (I) forms an acridinyl unit;
R, R^{a}, R^{b}, R^{c}, R³, R⁴ and R⁵ are each, independently of one another, unsubstituted or at least monosubstituted C₁-C₁₀-alkyl, C₃-C₁₀-cycloalkyl, C₃-C₁₀-heterocyclyl comprising at least one heteroatom selected from among N, 0 and S, C₅-C₁₀-aryl or C₅-C₁₀-heteroaryl comprising at least one heteroatom selected from among N, 0 and S,
where the substituents are selected from the group consisting of: F, Cl, Br, OH, CN, NH₂ and C₁-C₁₀-alkyl;
Y is a monoanionic ligand selected from the group consisting of H, F, Cl, Br, I, OCOR, OCOCF₃, OSO₂R, OSO₂CF₃, CN, OH, OR and N(R)₂ or uncharged molecules selected from the group consisting of NH₃, N(R)₃ and R₂NSO₂R;
X¹ represents one, two, three, four, five, six or seven substituents on one or more atoms of the acridinyl unit or one, two, three, four or five substituents on one or more atoms of the quinolinyl unit,
where the radicals X¹ are selected independently from the group consisting of hydrogen, F, Cl, Br, I, OH, NH₂, NO₂, - NC(O)R, C(O)NR₂, -OC(O)R, -C(O)OR, CN and borane derivatives which can be obtained from the catalyst of the formula (I) by reaction with NaBH₄ and unsubstituted or at least monosubstituted C₁-C₁₀-alkoxy, C₁-C₁₀-alkyl, C₃-C₁₀-cycloalkyl, C₃-C₁₀-heterocyclyl comprising at least one heteroatom selected from among N, 0 and S, C₅-C₁₀-aryl and C₅-C₁₀-heteroaryl comprising at least one heteroatom selected from among N, 0 and S,
where the substituents are selected from the group consisting of: F, Cl, Br, OH, CN, NH₂ and C₁-C₁₀-alkyl;
and
M is iron, cobalt, nickel, ruthenium, rhodium, palladium, osmium, iridium or platinum.

9. The process according to either claim 1 or 8, wherein the complex catalyst is a catalyst of the formula (XVI) :

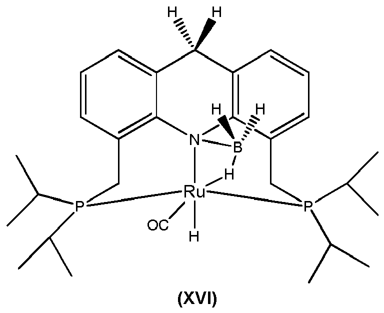

10. The process according to any of claims 1 to 5, wherein Y in the complex catalyst is selected from among F, Cl and Br.

11. The process according to any of claims 2 to 10, wherein L³ in the complex catalyst is CO.

12. The process according to claim 1, wherein the alcohol amination is carried out in the presence of at least one complex catalyst comprising at least one element selected from groups 8, 9 and 10 of the Periodic Table and also at least one phosphorus donor ligand of the general formula (XXI), 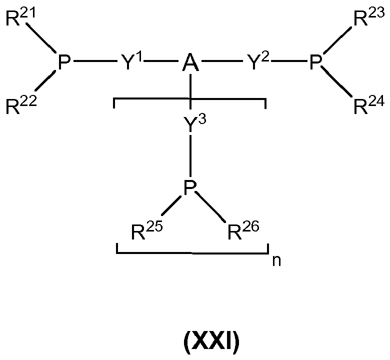 where
n is 0 or 1,
R²¹, R²², R²³, R²⁴, R²⁵, R²⁶ are each, independently of one another, unsubstituted or at least monosubstituted C₁-C₁₀-alkyl, C₁-C₄-alkyldiphenylphosphine (-C₁-C₄-alkyl-P (phenyl) ₂), C₃-C₁₀-cycloalkyl, C₃-C₁₀-heterocyclyl comprising at least one heteroatom selected from among N, 0 and S, C₅-C₁₄-aryl or C₅-C₁₀-heteroaryl comprising at least one heteroatom selected from among N, 0 and S,
where the substituents are selected from the group consisting of: F, Cl, Br, OH, CN, NH₂ and C₁-C₁₀-alkyl;
A is
i)a bridging group selected from the group consisting of unsubstituted or at least monosubstituted N, 0, P, C₁-C₆-alkane, C₃-C₁₀-cycloalkane, C₃-C₁₀-heterocycloalkane comprising at least one heteroatom selected from among N, O and S, C₅-C₁₄-aromatic and C₅-C₆-heteroaromatic comprising at least one heteroatom selected from among N, 0 and S,
where the substituents are selected from the group consisting of:
C₁-C₄-alkyl, phenyl, F, Cl, Br, OH, OR²⁷, NH₂, NHR²⁷ and N(R²⁷)₂,
where R²⁷ is selected from among C₁-C₁₀-alkyl and C₅-C₁₀-aryl;
or
ii) a bridging group of the formula (XXII) or (XXIII) : 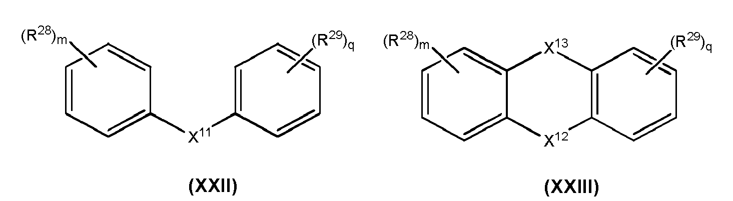
m, q are each, independently of one another, 0, 1, 2, 3 or 4,
R²⁸, R²⁹ are selected independently from the group consisting of C₁-C₁₀-alkyl, F, Cl, Br, OH, OR²⁷, NH₂, NHR²⁷ and N(R²⁷)₂,
where R²⁷ is selected from among C₁-C₁₀-alkyl and C₅-C₁₀-aryl;
X¹¹, X¹² are each, independently of one another, NH, 0 or S,
X¹³ is a bond, NH, NR³⁰, 0, S or CR³¹R³²;
R³⁰ is unsubstituted or at least monosubstituted C₁-C₁₀-alkyl, C₃-C₁₀-cycloalkyl, C₃-C₁₀-heterocyclyl comprising at least one heteroatom selected from among N, 0 and S, C₅-C₁₄-aryl or C₅-C₁₀-heteroaryl comprising at least one heteroatom selected from among N, 0 and S,
where the substituents are selected from the group consisting of: F, Cl, Br, OH, CN, NH₂ and C₁-C₁₀-alkyl;
R³¹, R³² are each, independently of one another, unsubstituted or at least monosubstituted C₁-C₁₀-alkyl, C₁-C₁₀-alkoxy, C₃-C₁₀-cycloalkyl, C₃-C₁₀-cycloalkoxy, C₃-C₁₀-heterocyclyl comprising at least one heteroatom selected from among N, 0 and S, C₅-C₁₄-aryl, C₅-C₁₄-aryloxy or C₅-C₁₀-heteroaryl comprising at least one heteroatom selected from among N, 0 and S,
where the substituents are selected from the group consisting of: F, Cl, Br, OH, CN, NH₂ and C₁-C₁₀-alkyl;
Y¹, Y², Y³ are each, independently of one another, a bond, unsubstituted or at least monosubstituted methylene, ethylene, trimethylene, tetramethylene, pentamethylene or hexamethylene,
where the substituents are selected from the group consisting of: F, Cl, Br, OH, OR²⁷, CN, NH₂, NHR²⁷, N(R²⁷)₂ and C₁-C₁₀-alkyl,
where R²⁷ is selected from among C₁-C₁₀-alkyl and C₅-C₁₀-aryl.

13. The process according to any of claims 1 to 11, wherein the diol has two functional groups of the formula (-CH₂-OH).

14. The process according to any of claims 1 to 13, wherein the preparation of the alcoholamines is carried out at a temperature of from 110 to 160°C and a pressure of from 1 to 15 MPa.

15. The use of a complex catalyst comprising at least one element selected from groups 8, 9 and 10 of the Periodic Table and also at least one phosphorus donor ligand for the homogeneously catalyzed preparation of alkanolamines which have a primary amino group (-NH₂) and a hydroxyl group (-OH) by alcohol amination of diols having two hydroxyl groups (-OH) by means of ammonia.

## Revendications

1. Procédé de fabrication d'alcanolamines comprenant un groupe amino primaire (-NH₂) et un groupe hydroxyle (-OH) par amination d'alcool de diols comprenant deux groupes hydroxyle (-OH) avec de l'ammoniac avec clivage d'eau, dans lequel la réaction est réalisée sous catalyse homogène en présence d'au moins un catalyseur complexe contenant au moins un élément choisi dans les groupes 8, 9 et 10 du tableau périodique, ainsi qu'au moins un ligand donneur de phosphore.

2. Procédé selon la revendication 1, dans lequel le catalyseur complexe est un catalyseur de formule (I) : 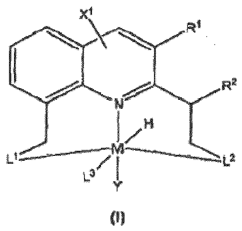 dans laquelle
L¹ et L² signifient indépendamment l'un de l'autre PR^{a}R^{b}, NR^{a}R^{b}, sulfure, SH, S(=O)R, hétéroaryle en C₅-C₁₀ contenant au moins un hétéroatome choisi parmi N, 0 et S, AsR^{a}R^{b}, SbR^{a}R^{b} ou des carbènes N-hétérocycliques de formule (II) ou (III) : 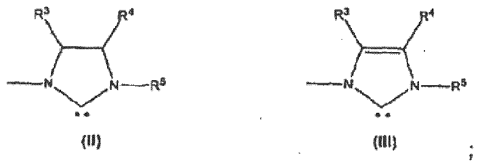 L³ signifie un donneur de deux électrons monodentate choisi dans le groupe constitué par CO, PR^{a}R^{b}R^{c}, NO⁺, AsR^{a}R^{b}R^{c}, SbR^{a}R^{b}R^{c}, SR^{a}R^{b}, RCN, RNC, N₂, PF₃, CS, pyridine, thiophène, tétrahydrothiophène et des carbènes N-hétérocycliques de formule (II) ou (III) ;
R¹ et R² signifient tous les deux hydrogène ou forment conjointement avec les atomes de carbone auxquels ils sont reliés un cycle phényle, qui forme conjointement avec l'unité quinolinyle de la formule (I) une unité acridinyle ;
R, R^{a}, R^{b}, R^{c}, R³, R⁴ et R⁵ signifient indépendamment les uns des autres alkyle en C₁-C₁₀, cycloalkyle en C₃-C₁₀, hétérocyclyle en C₃-C₁₀ contenant au moins un hétéroatome choisi parmi N, 0 et S, aryle en C₅-C₁₀ ou hétéroaryle en C₅-C₁₀ contenant au moins un hétéroatome choisi parmi N, 0 et S, non substitué ou au moins monosubstitué,
les substituants étant choisis dans le groupe constitué par : F, Cl, Br, OH, CN, NH₂ et alkyle en C₁-C₁₀ ;
Y signifie un ligand monoanionique choisi dans le groupe constitué par H, F, Cl, Br, I, OCOR, OCOCF₃, OSO₂R, OSO₂CF₃, CN, OH, OR et N(R)₂ ou des molécules neutres choisies dans le groupe constitué par NH₃, N(R)₃ et R₂NSO₂R ;
X¹ signifie un, deux, trois, quatre, cinq, six ou sept substituants sur un ou plusieurs atomes de l'unité acridinyle ou un, deux, trois, quatre ou cinq substituants sur un ou plusieurs atomes de l'unité quinolinyle,
les X¹ étant choisis indépendamment les uns des autres dans le groupe constitué par hydrogène, F, Cl, Br, I, OH, NH₂, NO₂, -NC(O)R, C(O)NR₂, -OC(O)R, -C(O)OR, CN et les dérivés de borane, qui peuvent être obtenus à partir du catalyseur de formule (I) par réaction avec NaBH₄, et alcoxy en C₁-C₁₀, alkyle en C₁-C₁₀, cycloalkyle en C₃-C₁₀, hétérocyclyle en C₃-C₁₀ contenant au moins un hétéroatome choisi parmi N, 0 et S, aryle en C₅-C₁₀ et hétéroaryle en C₅-C₁₀ contenant au moins un hétéroatome choisi parmi N, 0 et S, non substitué ou au moins monosubstitué,
les substituants étant choisis dans le groupe constitué par : F, Cl, Br, OH, CN, NH₂ et alkyle en C₁-C₁₀ ;
et
M signifie fer, cobalt, nickel, ruthénium, rhodium, palladium, osmium, iridium ou platine.

3. Procédé selon la revendication 1 ou 2, dans lequel R¹ et R² signifient tous les deux hydrogène et le catalyseur complexe est un catalyseur de formule (IV) : 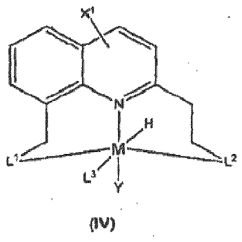 et X¹, L¹, L², L³ et Y ont les significations indiquées dans la revendication 2.

4. Procédé selon la revendication 1 ou 2, dans lequel R¹ et R² forment conjointement avec les atomes de carbone auxquels ils sont reliés un cycle phényle, qui forme conjointement avec l'unité quinolinyle de la formule (I) une unité acridinyle, et le catalyseur complexe est un catalyseur de formule (V) : 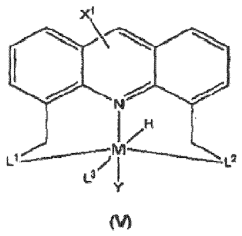 dans laquelle X¹, L¹, L², L³ et Y ont les significations indiquées dans la revendication 2.

5. Procédé selon la revendication 1 ou 2, dans lequel le catalyseur complexe est choisi dans le groupe des catalyseurs de formule (VI), (VII), (VIII), (IX), (X), (XI), (XII) et (XIII) : 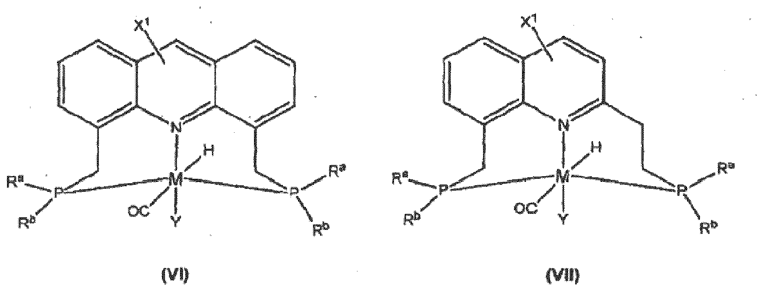 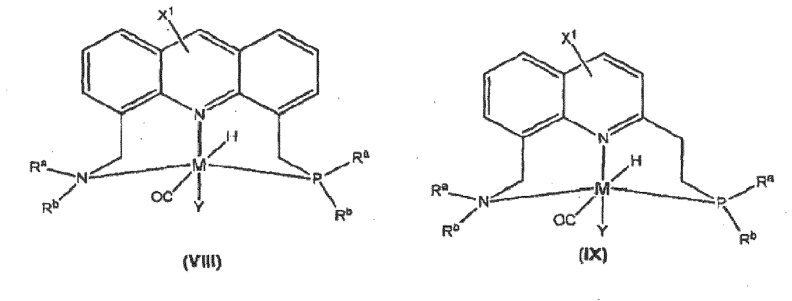 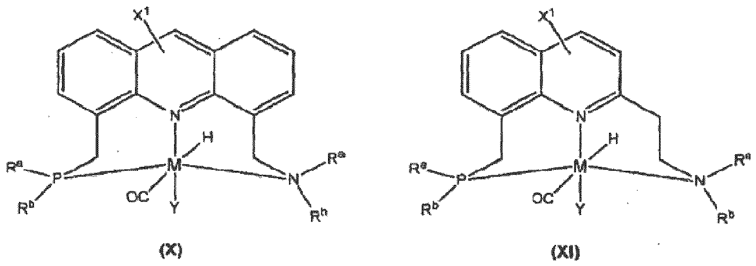 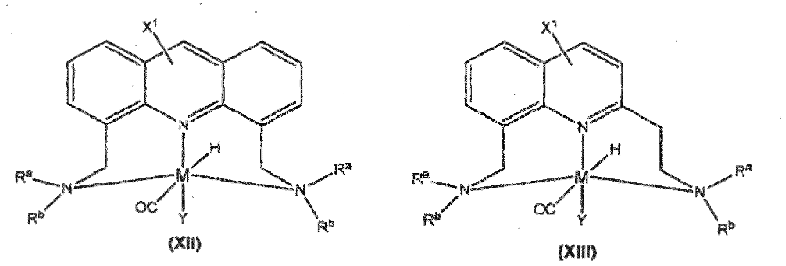 et X¹, R^{a}, R^{b} et Y ont les significations indiquées dans la revendication 2.

6. Procédé selon la revendication 1 ou 2, dans lequel le catalyseur complexe est un catalyseur de formule (XIVa) :

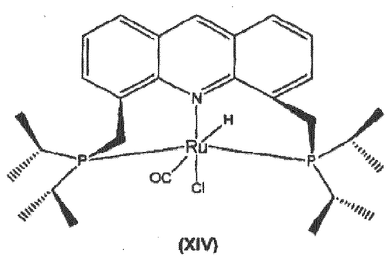

7. Procédé selon la revendication 1 ou 2, dans lequel le catalyseur complexe est un catalyseur de formule (XIVb) :

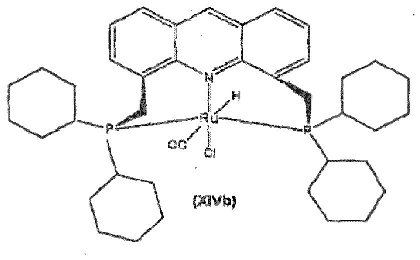

8. Procédé selon la revendication 1, dans lequel le catalyseur complexe est un catalyseur de formule (XV) : 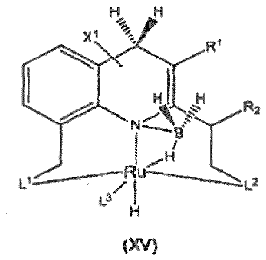 dans laquelle
L¹ et L² signifient indépendamment l'un de l'autre PR^{a}R^{b}, NR^{a}R^{b}, sulfure, SH, S(=O)R, hétéroaryle en C₅-C₁₀ contenant au moins un hétéroatome choisi parmi N, 0 et S, AsR^{a}R^{b}, SbR^{a}R^{b} ou des carbènes N-hétérocycliques de formule (II) ou (III) : 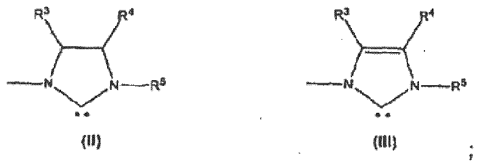 L³ signifie un donneur de deux électrons monodentate choisi dans le groupe constitué par CO, PR^{a}R^{b}R^{c}, NO⁺, AsR^{a}R^{b}R^{c}, SbR^{a}R^{b}R^{c}, SR^{a}R^{b}, RCN, RNC, N₂, PF₃, CS, pyridine, thiophène, tétrahydrothiophène et des carbènes N-hétérocycliques de formule (II) ou (III) ;
R¹ et R² signifient tous les deux hydrogène ou forment conjointement avec les atomes de carbone auxquels ils sont reliés un cycle phényle, qui forme conjointement avec l'unité quinolinyle de la formule (I) une unité acridinyle ;
R, R^{a}, R^{b}, R^{c}, R³, R⁴ et R⁵ signifient indépendamment les uns des autres alkyle en C₁-C₁₀, cycloalkyle en C₃-C₁₀, hétérocyclyle en C₃-C₁₀ contenant au moins un hétéroatome choisi parmi N, 0 et S, aryle en C₅-C₁₀ ou hétéroaryle en C₅-C₁₀ contenant au moins un hétéroatome choisi parmi N, 0 et S, non substitué ou au moins monosubstitué,
les substituants étant choisis dans le groupe constitué par : F, Cl, Br, OH, CN, NH₂ et alkyle en C₁-C₁₀ ;
Y signifie un ligand monoanionique choisi dans le groupe constitué par H, F, Cl, Br, I, OCOR, OCOCF₃, OSO₂R, OSO₂CF₃, CN, OH, OR et N (R)₂ ou des molécules neutres choisies dans le groupe constitué par NH₃, N(R)₃ et R₂NSO₂R ;
X¹ signifie un, deux, trois, quatre, cinq, six ou sept substituants sur un ou plusieurs atomes de l'unité acridinyle ou un, deux, trois, quatre ou cinq substituants sur un ou plusieurs atomes de l'unité quinolinyle,
les X¹ étant choisis indépendamment les uns des autres dans le groupe constitué par hydrogène, F, Cl, Br, I, OH, NH₂, NO₂, -NC(O)R, C(O)NR₂, -OC(O)R, -C(O)OR, CN et les dérivés de borane, qui peuvent être obtenus à partir du catalyseur de formule (I) par réaction avec NaBH₄, et alcoxy en C₁-C₁₀, alkyle en C₁-C₁₀, cycloalkyle en C₃-C₁₀, hétérocyclyle en C₃-C₁₀ contenant au moins un hétéroatome choisi parmi N, 0 et S, aryle en C₅-C₁₀ et hétéroaryle en C₅-C₁₀ contenant au moins un hétéroatome choisi parmi N, 0 et S, non substitué ou au moins monosubstitué,
les substituants étant choisis dans le groupe constitué par : F, Cl, Br, OH, CN, NH₂ et alkyle en C₁-C₁₀ ;
et
M signifie fer, cobalt, nickel, ruthénium, rhodium, palladium, osmium, iridium ou platine.

9. Procédé selon l'une quelconque des revendications 1 ou 8, dans lequel le catalyseur complexe est un catalyseur de formule (XVI) :

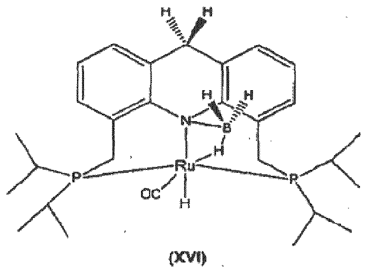

10. Procédé selon l'une quelconque des revendications 1 à 5, dans lequel Y dans le catalyseur complexe est choisi parmi F, Cl et Br.

11. Procédé selon l'une quelconque des revendications 2 à 10, dans lequel L³ dans le catalyseur complexe représente CO.

12. Procédé selon la revendication 1, dans lequel l'amination d'alcool est réalisée en présence d'au moins un catalyseur complexe qui contient au moins un élément choisi dans les groupes 8, 9 et 10 du tableau périodique, ainsi qu'au moins un ligand donneur de phosphore de formule générale (XXI) 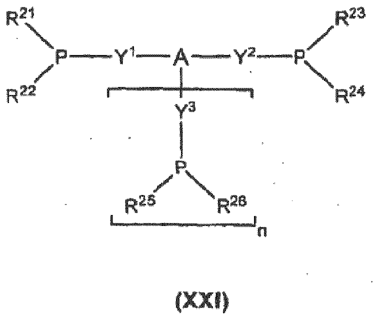 dans laquelle
n représente 0 ou 1,
R²¹, R²², R²³, R²⁴, R²⁵, R²⁶ représentent indépendamment les uns des autres alkyle en C₁-C₁₀, alkyle en C₁-C₄-diphénylphosphine (alkyle en C₁-C₄-P (phényl) ₂), cycloalkyle en C₃-C₁₀, hétérocyclyle en C₃-C₁₀ contenant au moins un hétéroatome choisi parmi N, 0 et S, aryle en C₅-C₁₄ ou hétéroaryle en C₅-C₁₀ contenant au moins un hétéroatome choisi parmi N, 0 et S, non substitué ou au moins monosubstitué,
les substituants étant choisis dans le groupe constitué par : F, Cl, Br, OH, CN, NH₂ et alkyle en C₁-C₁₀ ;
A représente
i) un groupe de pontage choisi dans le groupe constitué par N, 0, P, alcane en C₁-C₆, cycloalcane en C₃-C₁₀, hétérocycloalcane en C₃-C₁₀ contenant au moins un hétéroatome choisi parmi N, 0 et S, aromatique en C₅-C₁₄ ou hétéroaromatique en C₅-C₆ contenant au moins un hétéroatome choisi parmi N, 0 et S, non substitué ou au moins monosubstitué,
les substituants étant choisis dans le groupe constitué par : alkyle en C₁-C₄, phényle, F, Cl, Br, OH, OR²⁷, NH₂, NHR²⁷ ou N(R²⁷)₂,
R²⁷ étant choisi parmi alkyle en C₁-C₁₀ et alkyle en C₅-C₁₀ ;
ou
ii) un groupe de pontage de formule (XXII) ou (XXIII) : 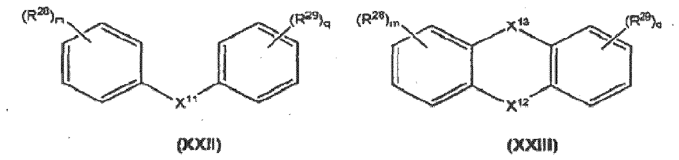
m, q représentent indépendamment l'un de l'autre 0, 1, 2, 3 ou 4,
R²⁸, R²⁹ sont choisis indépendamment l'un de l'autre dans le groupe constitué par alkyle en C₁-C₁₀, F, Cl, Br, OH, OR²⁷, NH₂, NHR²⁷ et N(R²⁷)₂,
R²⁷ étant choisi parmi alkyle en C₁-C₁₀ et aryle en C₅-C₁₀ ;
X¹¹, X¹² représentent indépendamment l'un de l'autre NH, O ou S,
X¹³ représente une liaison, NH, NR³⁰, 0, S ou CR³¹R³², R³⁰ représente alkyle en C₁-C₁₀, cycloalkyle en C₃-C₁₀, hétérocyclyle en C₃-C₁₀ contenant au moins un hétéroatome choisi parmi N, 0 et S, aryle en C₅-C₁₄ ou hétéroaryle en C₅-C₁₀ contenant au moins un hétéroatome choisi parmi N, 0 et S, non substitué ou au moins monosubstitué,
les substituants étant choisis dans le groupe constitué par : F, Cl, Br, OH, CN, NH₂ et alkyle en C₁-C₁₀ ;
R³¹, R³² représentent indépendamment l'un de l'autre alkyle en C₁-C₁₀, alcoxy en C₁-C₁₀, cycloalkyle en C₃-C₁₀, cycloalcoxy en C₃-C₁₀, hétérocyclyle en C₃-C₁₀ contenant au moins un hétéroatome choisi parmi N, 0 et S, aryle en C₅-C₁₄, aryloxy en C₅-C₁₄ ou hétéroaryle en C₅-C₁₀ contenant au moins un hétéroatome choisi parmi N, 0 et S, non substitué ou au moins monosubstitué,
les substituants étant choisis dans le groupe constitué par : F, Cl, Br, OH, CN, NH₂ et alkyle en C₁-C₁₀ ;
Y¹, Y², Y³ représentent indépendamment les uns des autres une liaison, méthylène, éthylène, triméthylène, tétraméthylène, pentaméthylène ou hexaméthylène non substitué ou au moins monosubstitué,
les substituants étant choisis dans le groupe constitué par : F, Cl, Br, OH, OR²⁷, CN, NH₂, NHR²⁷, N(R²⁷)₂ et alkyle en C₁-C₁₀,
R²⁷ étant choisi parmi alkyle en C₁-C₁₀ et aryle en C₅-C₁₀.

13. Procédé selon l'une quelconque des revendications 1 à 11, dans lequel le diol comprend deux groupes fonctionnels de formule (-CH₂-OH).

14. Procédé selon l'une quelconque des revendications 1 à 13, dans lequel la fabrication des alcanolamines est réalisée à une température de 110 à 160 °C et à une pression de 1 à 15 MPa.

15. Utilisation d'un catalyseur complexe qui contient au moins un élément choisi dans les groupes 8, 9 et 10 du tableau périodique, ainsi qu'au moins un ligand donneur de phosphore, pour la fabrication sous catalyse homogène d'alcanolamines comprenant un groupe amino primaire (-NH₂) et un groupe hydroxyle (-OH), par amination d'alcool de diols comprenant deux groupes hydroxyle (-OH) avec de l'ammoniac.
